(19) Europäisches Patentamt
European Patent Office
Office européen des brevets

(11) **EP 3 916 105 B1**

(12) **EUROPEAN PATENT SPECIFICATION**

(45) Date of publication and mention
of the grant of the patent:
**25.01.2023 Bulletin 2023/04**

(21) Application number: **19941307.1**

(22) Date of filing: **14.08.2019**

(51) International Patent Classification (IPC):
*C12Q 1/68* (2018.01)     *C12M 1/34* (2006.01)
*C12Q 1/6809* (2018.01)     *G16B 20/20* (2019.01)
*G16B 30/00* (2019.01)     *C12Q 1/6883* (2018.01)

(52) Cooperative Patent Classification (CPC):
(C-Sets available)
**G16B 20/20; C12Q 1/6809; G16B 30/00;**
C12Q 1/6883; C12Q 2600/156          (Cont.)

(86) International application number:
**PCT/CN2019/100629**

(87) International publication number:
**WO 2021/026828 (18.02.2021 Gazette 2021/07)**

(54) **METHOD AND DEVICE FOR DETERMINING FETAL NUCLEIC ACID CONCENTRATION IN BLOOD OF PREGNANT WOMAN**

VERFAHREN UND VORRICHTUNG ZUR BESTIMMUNG DER FETALEN NUKLEINSÄUREKONZENTRATION IM BLUT EINER SCHWANGEREN FRAU

PROCÉDÉ ET DISPOSITIF POUR DÉTERMINER LA CONCENTRATION D'ACIDE NUCLÉIQUE FOETAL DANS LE SANG D'UNE FEMME ENCEINTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.12.2021 Bulletin 2021/48**

(73) Proprietor: **BGI Genomics Co., Limited Guangdong 518083 (CN)**

(72) Inventors:
  • **CHEN, Ruoyan**
    **Shenzhen, Guangdong 518083 (CN)**
  • **JIN, Xin**
    **Shenzhen, Guangdong 518083 (CN)**
  • **JU, Jia**
    **Shenzhen, Guangdong 518083 (CN)**
  • **LIU, Siyang**
    **Shenzhen, Guangdong 518083 (CN)**

(74) Representative: **DREISS Patentanwälte PartG mbB
Friedrichstraße 6
70174 Stuttgart (DE)**

(56) References cited:
WO-A1-2014/180910     WO-A1-2016/084079
CN-A- 102 753 703       CN-A- 104 120 181
CN-A- 104 232 777       CN-A- 105 189 787
CN-A- 107 133 491       JP-A- 2017 192 383
US-A1- 2002 137 088     US-A1- 2004 146 883

• JU JIA ET AL: "Estimation of cell-free fetal DNA fraction from maternal plasma based on linkage disequilibrium information", NPJ GENOMIC MEDICINE, vol. 6, no. 1, 12 October 2021 (2021-10-12), XP055895352, DOI: 10.1038/s41525-021-00247-z Retrieved from the Internet: URL:https://www.nature.com/articles/s41525 -021-00247-z>

(52) Cooperative Patent Classification (CPC): (Cont.)

C-Sets
**C12Q 1/6809, C12Q 2537/165**

**Description**

**FIELD**

The present disclosure relates to the field of gene detection, and particularly, to a method and a device for determining a fetal nucleic acid concentration in maternal blood.

**BACKGROUND**

Since the discovery of fetal cell-free DNA in maternal plasma in 1997, noninvasive prenatal testing (NIPT), which can obtain fetal DNA information by extracting circulating cell-free DNA (cfDNA) from the maternal plasma, has made great progress. It has been confirmed that a concentration of fetal cfDNA in maternal plasma cfDNA increases with an increase of the gestational age of blood collection, and also varies in different pregnant women. The accurate estimation of the concentration of the fetal cfDNA in maternal plasma cfDNA not only helps to enhance an accuracy of the NIPT, but also facilitates the study of its influence on various pregnancy complications and maternal phenotypes.

Many institutions have successively proposed methods for estimating a fetal nucleic acid concentration through different data and different methods. These methods can be classified into six categories: 1) a Y chromosome depth based method, which calculates the fetal nucleic acid concentration based on the principle that cfDNA sequencing reads derived from pregnant women in plasma cannot be mapped to a non-homologous region of Y chromosome of the human reference genome, and the reads that can be uniquely mapped to the non-homologous region of the Y chromosome of the human reference genome are all derived from the cfDNA of the male fetuses; 2) a single nucleotide polymorphism (SNP) method assisted by capture sequencing data, which utilizes homozygous sites but different in paternal and maternal genome and incorporate the reads depth information of maternal plasma to calculate the fetal nucleic acid concentration; 3) a sequencing reads depth distribution method, which divides a genome into windows (for example, 50kb), calculates the total number of reads of cfDNA in maternal plasma and a proportion of short reads in each window, uses the calculated results as input data to establish a regression model, training the model by the fetal cfDNA concentration calculated by Y chromosome depth as the true set, and using data of a super-large sample size, and then detecting the fetal cfDNA concentration using this trained model; 4) a methylation data method, in which the calculation is based on the differences in the methylation of DNA derived from different individuals or from different tissues of the same individual; 5) a cfDNA fragment length method, which estimates a concentration of fetal cfDNA by calculating a proportion of cfDNA fragments in the pregnant woman's body based on the known fact that the length of fetal cfDNA fragments in the maternal plasma have more proportion distribution between147bp and 167bp, and the length of cfDNA of pregnant women generally distribute between 167bp to 187bp; and 6) a nucleosome arrangement method, which estimates a concentration of fetal cfDNAbased on the difference in fragment length distribution of pregnant woman cfDNA and fetal cfDNA caused by different degradation degrees. Among the six methods, only methods 1), 3) and 6) can calculate fetal cfDNA concentration merely based on NIPT data, method 1) is limited to the calculation specified to male fetuses, and methods 3) and 6) can only be used for calculation of NIPT data of higher depths.

Currently, no method is capable to calculate the cfDNA concentration of both male and female fetuses indiscriminately based on ultra-low depth NIPT data.

**SUMMARY**

The present disclosure aims to solve one of the technical problems in the related art at least to a certain extent. In this regard, an object of the present disclosure is to provide a method and a device for determining a fetal nucleic acid concentration in maternal plasma.

In the course of long-term research, the applicant has the following findings.

As for the existing methods for calculating the fetal nucleic acid concentration, when detecting a fetal nucleic acid concentration in blood of a pregnant woman, other types of data in addition to NIPT data of blood plasma of the pregnant woman are required for assistance in most cases. Among them, 1) the single nucleotide polymorphism (SNP) method assisted by capture sequencing data requires the use of parental capture sequencing data or high-depth cfDNA sequencing data as an assistance, the calculation of fetal nucleic acid concentration can be completed only on basis of additionally obtaining the accurate genotypes of the father and mother (or at least the mother); 2) the methylation data method requires additional methylation data of the father and mother for the calculation of fetal nucleic acid concentration. These methods require different types of auxiliary data, which on the one hand, increases the difficulty of sampling (for example, requiring to obtain a father's blood sample), and on the other hand, increases the cost of analysis.

However, the methods that only require NIPT data of maternal plasma have additional requirements for the type of NIPT data and the sequencing depth. Among them, 1) the cfDNA fragment length calculation method requires accurate estimation of cfDNA fragment length, and thus only the paired-end sequencing (PE) can be adopted; and 2) the nucleosome arrangement calculation method requires the use of a depth difference of reads in the nucleosome unit and thus has certain requirements for the depth of NIPT sequencing, and thus it is impossible to only use the data of about 0.1x depth that is at present common in NIPT detection.

The remaining two methods, which require no additional data of other types as assistance and is not specific to NIPT data, both have their own limitations in application and cannot cover all NIPT samples. Among them, 1) the Y chromosome depth calculation method can only calculate the fetal nucleic acid concentration of male fetuses, but cannot calculate the fetal nucleic acid concentration of female fetuses; and 2) the sequencing reads depth distribution calculation method can only be used to estimate samples with a high fetal nucleic acid concentration, and cannot be applied to samples with a fetal nucleic acid concentrations smaller than or equal to 5%.

Therefore, it is still necessary to further improve the method for calculating a cfDNA concentration of both male and female fetuses indiscriminately by means of low-depth NIPT data. In this regard, the present disclosure provides a method and a device for determining a fetal nucleic acid concentration in maternal blood. The method or device can use only the NIPT data of maternal plasma to determine the fetal nucleic acid concentration, without requiring the assistance of other data. In addition, the method or device can be applied to the sequencing data of ultra-low depth (for example, about 0.1x), and has no limitations on the types of sequencing, and both the paired-end sequencing and the single-end sequencing can be employed; and the method or device is not specific to the sample types, and is applicable for both samples involving male fetuses and samples involving female fetuses.

Specifically, the present disclosure provides the following technical solutions.

According to a first aspect of the present disclosure, the present disclosure provides a method for determining a fetal nucleic acid concentration in maternal blood. The method includes: (1) determining, by a processor, first genotype information based on alignment of sequencing data with at least a part of a reference genome, the sequencing data being derived from a nucleic acid sample of the maternal blood; (2) correcting, by the processor, the first genotype information based on reference data by using a linkage disequilibrium relationship to obtain second genotype information; and (3) determining, by the processor, the fetal nucleic acid concentration based on a difference between the first genotype information and the second genotype information.

The present disclosure provides a method for determining a fetal nucleic acid concentration in maternal blood. In the method, first genotype information is obtained by alignment of sequencing data with a reference genome; the sequencing data are obtained by sequencing the nucleic acid sample of the maternal bloods, and the sequencing data includes fetal nucleic acid information and maternal nucleic acid information. The sequencing data also indirectly includes paternal nucleic acid information, since a part of the fetal nucleic acid information is derived from the father. The linkage disequilibrium relationship is utilized to correct the obtained first genotype information. In other words, the part of the nucleic acid information derived from the paternal parent is mixed in the sequencing data, and is derived from a different individual from the maternal information, and thus is corrected to a certain extent to obtain the second genotype information after correction. Then, a part of corrected genotype information can be determined by the difference between the first genotype information and the second genotype information, and the more the part of corrected genotype information, the higher the fetal cfDNA concentration in the maternal plasma. The fetal nucleic acid concentration can be determined based on the relationship between a proportion of the corrected genotype and the fetal cfDNA concentration in the maternal plasma.

The method for determining the fetal nucleic acid concentration in maternal blood provided by the present disclosure has many advantages in the following aspects: 1) throughout the method, only the NIPT data of maternal plasma is used without requiring the assistance of other data; 2) the method can be applied to the sequencing data of ultra-low depth (for example, about 0.1x), because that the linkage disequilibrium relationship is used to estimate the changes in the genome genotype, the changes in the genome genotype can reflect the fetal nucleic acid concentration as long as the data derived from two sources (pregnant woman and fetus) are mixed, even if the sequencing depth is low; 3) the method is not specific to the types of sequencing, and both the paired-end sequencing or the single-end sequencing are applicable; 4) the method is not specific to the sample types (both samples involving male fetuses and samples involving female fetuses are applicable), and has no limitation for the fetal nucleic acid concentration.

The method provided in the present disclosure breaks through the limitations of the sequencing depth, data type, and fetal gender of the estimated data has universality, requires no additional sampling and sequencing costs, and has extremely high application value in the NIPT field.

According to the embodiments of the present disclosure, the above-mentioned method for determining the fetal nucleic acid concentration in maternal blood may further include the following technical features.

In some embodiments of the present disclosure, the sequencing data are obtained by sequencing the nucleic acid sample of the maternal blood, and a depth of the sequencing may be 10X, 5X, 1X, 0.5X, 0.2X, or 0.1X. The nucleic acid sample of the maternal blood include fetal nucleic acid information and maternal nucleic acid information, and a part of the fetal nucleic acid information is derived from the paternal parent. Therefore, even the sequencing data obtained by sequencing the nucleic acid sample of the maternal blood are sequencing data with a low sequencing depth, e.g., sequencing data of up to 10X, up to 5X, up to 1X, or even 0.1X, the sequence data can be analyzed by the method provided in the present disclosure to determine the fetal nucleic acid concentration.

In some embodiments of the present disclosure, the sequencing data is obtained through the second-generation sequencing technology or the third-generation sequencing technology. The second-generation sequencing technology is also referred to as high-throughput sequencing technology, which can obtain many sequences one time. For example, the nucleic acid can be randomly broken into small fragments (about 250bp to 300bp) by physical or chemical means, and then these small molecule fragments are enriched by constructing a library and sequenced in a sequencer. The sequencer has regions where these fragments can be attached, and each fragment has an independent attachment region, such that the information of all attached DNA sequences can be detected in one time. The second-generation sequencing technology can detect a large number of sequences in one time, but the fragments are limited to, for example, about 250bp to 300bp, and the cost is relatively high. The commonly used second-generation sequencing technology can be Roche/454's pyrosequencing method for sequencing, or Illumina's fluorescent sequencing detection, ABI/Solid's fluorescent sequencing detection, or MGI's DNB sequencing detection, etc. The third-generation sequencing technology can make the sequencing length up to about 10KB and does not rely on PCR amplification. The third-generation sequencing technology allows a sequencing length up to about 10KB and does not rely on PCR amplification, for example, using PacBio's SMRT or single-molecule nanopore DNA sequencing technology developed by Oxford Nanopore Technologies. The sequencing data of the nucleic acid sample of maternal blood, whether obtained by the second-generation sequencing technology or the third-generation sequencing technology, or obtained by the single-end sequencing or the paired-end sequencing, can be analyzed according to the method provided in the present disclosure to determine the fetal nucleic acid concentration.

In some embodiments of the present disclosure, the reference genome includes at least one strong linkage region in the human genome. The "strong linkage region" varies with the size and structure of the studied population, and is generally defined as a region in which a probability of historical recombination between any pair of variation sites is less than 5%. The reference genome may contain one strong linkage region, two strong linkage regions, three strong linkage regions, or even more. Generally speaking, regardless of cost, the more the strong linkage regions contained in the reference genome, the more accurate the fetal nucleic acid concentration in the maternal blood finally determined after alignment and calculation is.

When selecting strong linkage regions, all or part of the strong linkage regions of the population in the genome can be found, depending on the size and structure of the studied population, and then based on the ranges of these strong linkage regions, strong linkage regions with appropriate sizes can be selected as the reference genome. Generally speaking, the greater the number and proportion of variation sites of the maternal DNA and fetal DNA covered in the selected strong linkage region, the more accurate the fetal nucleic acid concentration in the maternal blood calculated using the reference genome containing this strong linkage region is. In some embodiments of the present disclosure, a length of the strong linkage region ranges from 5 mb to 10 mb, for example, 10 mb, 9 mb, 8 mb, 7 mb, 6 mb, or 5 mb. The above-listed lengths of the strong linkage region may have a variation of 10% to 20%, for example, the length of the strong linkage region can be 10 mb, 11 mb or 12 mb, 9 mb or 8 mb, etc. In this way, the information derived from paternal sequencing data can be accurately corrected.

In some embodiments of the present disclosure, the first genotype information is determined based on a support number of sequencing reads. When the first genotype information is obtained, the first genotype information is determined based on the support number of sequencing reads. For example, for a certain site, if 100 sequencing reads support base A, 8 sequencing reads support base G and 20 sequencing reads support base T, then the base at this site is determined to be base A. In this way, the genotype information of each site can be obtained, and the required first genotype information can be determined by aligning to at least a part of the reference genome.

In some embodiments of the present disclosure, the first genotype information includes at least one of SNV or Indel. The first genotype information includes single nucleotide variation (SNV) information and/or small fragment insertion-deletion (Indel) information, and the paternal nucleic acid information can be reflected through the correction of these information, thereby realizing accurate determination of the fetal nucleic acid concentration.

In some embodiments of the present application, the reference data includes multiple pieces of variation site information and multiple pieces of variation frequency information. Using data containing the multiple of pieces of variation site information and the multiple of pieces of variation frequency information as the reference data, the first genotype information can be corrected based on the linkage disequilibrium relationship, so as to correct some of sequencing information from the father, and then the fetal nucleic acid concentration is determined based on a correlation between the corrected information and the fetal nucleic acid concentration.

In some embodiments of the present disclosure, the correction is performed through IMPUTE2. IMPUTE2, as an imputation algorithm, which is actually a genotype completion and correction algorithm for sites with deletion data or low accuracy. In addition to IMPUTE2, other imputation methods can also be used to perform the site correction by using LD information, such as BNEAGLE, PHASE and other software.

In some embodiments of the present disclosure, the step (3) further includes: step (3-1) of determining a difference ratio between the first genotype information and the second genotype information; and step (3-2) of determining the fetal nucleic acid concentration based on the difference ratio obtained in step (3-1) and a pre-trained formula, the formula being determined based on a plurality of training samples with known fetal nucleic acid concentrations. In view of a relationship between the plurality of known fetal nucleic acid concentrations and the difference ratio of the first genotype information and the second genotype information, different formulas or models, for example, the linear regression model, or other models that effectively integrate all information, such as the random forest model or other deep learning models, etc., are used for training to correlate the fetal nucleic acid concentration with the difference ratio. After calculating the difference ratio between the first genotype information and the second genotype information of a nucleic acid sample from a given maternal blood, the fetal nucleic acid concentration in this maternal blood can be determined by the pre-trained formula. That is, in the early stage, some samples with known fetal nucleic acid concentrations can be used as a training set that is fitted with the help of the method provided by the present disclosure using different formulas or models so as to determine the formula; and in the subsequent application, the fetal cfDNA concentration of one or more samples can be predicted without requiring additional samples with known fetal nucleic acid concentrations.

In some embodiments of the present disclosure, the term "plurality of" indicates at least 100, for example, 100, 500, 1,000, 5,000, or more. When a formula or model is used for training, the greater the number of training samples, the more accurate the formula, and the more accurate the fetal nucleic acid concentration in the maternal blood measured by the formula. Of course, too many samples can also increase the calculation cost and the cost from the sample size itself. When both the cost and training accuracy are optimal, the number of these training samples can range from 5,000 to 1,0000, for example, 5,000, 6,000, 7,000, 8,000, 9,000, or 10,000, which is not limited herein.

According to a second aspect of the present disclosure, the present disclosure provides a device for determining a fetal nucleic acid concentration in maternal blood. The device can determine the fetal nucleic acid concentration in the maternal blood by using only the NIPT data of maternal plasma without the assistance of other data. In addition, the device can be applied to the sequencing data of ultra-low depth. Besides, the device has no limitations on the types of samples, and are applicable for both samples bearing male fetuses or samples bearing female fetuses. The device includes: an alignment unit configured to determine a first genotype information based on alignment between sequencing data and at least a part of a reference genome, in which the sequencing data are derived from a nucleic acid sample of the maternal blood; a correction unit connected to the alignment unit and configured to correct the first genotype information based on reference data by using a linkage disequilibrium relationship to obtain second genotype information; and a calculation unit connected to the alignment unit and the correction unit and configured to determine the fetal nucleic acid concentration based on a difference between the first genotype information and the second genotype information.

According to the embodiments of the present disclosure, the above device for determining the fetal nucleic acid concentration in the maternal blood can further have the following technical features. These technical features are mentioned or involved in the above-mentioned method for determining the fetal nucleic acid concentration in the maternal blood, and the functions of the features are similar to those in the above-mentioned method for determining the fetal nucleic acid concentration in maternal blood, and will not be described in detail herein.

In some embodiments of the present disclosure, in the device, the sequencing data are obtained by sequencing the nucleic acid sample of the maternal blood, and a depth of the sequencing may be 10X, 5X, 1X, 0.5X, 0.2X, or 0.1X. That is, the device provided by the present disclosure can use not only high-depth or relatively-high-depth sequencing data to determine the fetal nucleic acid concentration in the maternal blood, but also low-depth sequencing or ultra-low-depth sequencing data to determine the fetal nucleic acid concentration in the maternal blood.

In some embodiments of the present disclosure, in the device, the sequencing data are obtained by means of the second-generation sequencing technology or the third-generation sequencing technology.

In some embodiments of the present disclosure, in the device, the reference genome includes at least one strong linkage region in the human genome.

In some embodiments of the present disclosure, in the device, a length of the strong linkage region ranges from 5mb to 10mb, for example, 10mb, 9mb, 8mb, 7mb, 6mb, or 5mb. The length of the strong linkage region is not specifically limited in the present disclosure.

In some embodiments of the present disclosure, in the device, the first genotype information is determined based on a support number of sequencing reads.

In some embodiments of the present disclosure, in the device, the first genotype information includes at least one of SNV or Indel.

In some embodiments of the present disclosure, in the device, the reference data includes a plurality of pieces of variation site information and a plurality of pieces of variation frequency information.

In some embodiments of the present disclosure, in the device, the correction is performed through IMPUTE2.

In some embodiments of the present disclosure, the calculation unit further includes: a difference ratio calculation unit configured to determine a difference ratio between the first genotype information and the second genotype information; and a fetal nucleic acid concentration calculation unit connected to the difference ratio calculation unit. The fetal nucleic acid concentration calculation unit is configured to determine the fetal nucleic acid concentration based on the difference ratio obtained in the difference ratio calculation unit and a pre-trained formula. The training formula is determined based on a plurality of training samples with known fetal nucleic acid concentrations.

In some embodiments of the present disclosure, the term "plurality of" may indicate 5,000 to 10,000, for example, 5,000, 6,000, 7,000, 8,000, 9,000, or 10,000. The number of samples is not specifically limited herein.

According to a third aspect of the present disclosure, the present disclosure provides a computer device, including a memory, a processor, and a computer program stored on the memory and executable on the processor. The program, when executed by the processor, implements the method according to any one of embodiments of the first aspect of the present disclosure. Therefore, only the NIPT data of maternal plasma is required, and the fetal nucleic acid concentration can be quickly determined with the aids of the linkage disequilibrium relationship, and it can be applied to low-depth sequencing data, being not specific to the fetal nucleic acid concentration and the sample type.

According to a fourth aspect of the present disclosure, the present disclosure provides a computer readable storage medium having a computer program stored thereon. The program, when executed by a processor, implements the method according to any one of the embodiments of the first aspect of the present disclosure. Therefore, only the NIPT data of maternal plasma is required, and the fetal nucleic acid concentration can be quickly determined with the aids of the linkage disequilibrium relationship, and it can be applied to low-depth sequencing data, not specific to the fetal nucleic acid concentration and the sample type.

## BRIEF DESCRIPTION OF DRAWINGS

FIG. 1 is a structural schematic diagram of a device for determining a fetal nucleic acid concentration in maternal blood according to an embodiment of the present disclosure.

FIG. 2 is a structural schematic diagram of a calculation unit in a device for determining a fetal nucleic acid concentration in maternal blood according to an embodiment of the present disclosure.

FIG. 3 is a schematic diagram of a method for determining a fetal nucleic acid concentration through model prediction according to an embodiment of the present disclosure.

FIG. 4 is a graph illustrating prediction results of fetal nucleic acid concentrations of a 1000-sample test data set in accordance with a model obtained by using 10,000 samples as a training set according to an embodiment of the present disclosure.

FIG. 5 is a graph illustrating prediction results of fetal nucleic acid concentrations of a 1000-sample test data set in accordance with a model obtained by using 10,000 samples as a training set according to an embodiment of the present disclosure.

## DESCRIPTION OF EMBODIMENTS

Embodiments of the present disclosure are described in detail below. Examples of the embodiments are illustrated in the accompanying drawings, throughout which the identical or similar reference sign indicates the identical or similar elements or elements with the same or similar functions. The embodiments described below with reference to the accompanying drawings are illustrative and intended to explain the present disclosure, but should not be construed as limiting the present disclosure.

Meanwhile, in order to facilitate the understanding of those skilled in the art, some terms of the present disclosure are explained and described. It should be noted that these explanations and descriptions are merely intended to help the understanding of the technical solutions of the present disclosure, but should not be regarded as limitations of the protection scope of the present disclosure.

Herein, the terms "first genotype information" and "second genotype information" refer to information containing the genotype of each site, and represents the original genotypes obtained from the sequencing data and the genotypes after correction using linkage disequilibrium information, respectively.

The term "linkage" is used to describe a relationship between two sites. If a distance between two or more sites is relatively small, there is a small probability that crossover occurs during meiosis and alleles on two sites of the same chromosome are separated. That is, the alleles at the two sites are not independent when passed to the next generation, for example, the alleles at the two sites tend to be passed on together, which phenomenon is referred as to the linkage. A "strong linkage region" may vary according to the size and structure of the studied population, and is generally defined as a region in which a probability of historical recombination occurring between any pair of variation sites is less than 5%.

The linkage disequilibrium refers to a situation that a probability that a certain genotype combination of two variation sites is inherited at the same time is greater than a random probability. That is, as long as a certain genotype combination of two sites is not completely inherited independently, the linkage disequilibrium exists between the two sites.

According to a first aspect of the present disclosure, the present disclosure provides a method for determining a fetal nucleic acid concentration in maternal blood. The method includes: (1) determining first genotype information based on alignment of sequencing data with at least a part of a reference genome, where the sequencing data are derived from a nucleic acid sample of the maternal blood; (2) correcting the first genotype information based on reference data by using a linkage disequilibrium relationship, to obtain second genotype information; and (3) determining the fetal nucleic acid concentration based on a difference between the first genotype information and the second genotype information. With the method provided by the present disclosure, the fetal DNA concentration in maternal blood can be detected and determined.

When the linkage disequilibrium relationship is used for correction, the correction can be done based on existing methods or software. For example, the correction or rectification can be performed by means of Imputation. The Imputation is a method of genotype completion and correction for sites with deletion data or low accuracy. Specifically, the linkage disequilibrium (LD) relationship between the analyzed site and its nearby sites with higher accuracy is utilized to find the haplotype that best matches the analyzed site (by using haplotype information in the reference population, or using haplotype information among different individuals of the analyzed population), so as to infer the deletion genotype at the analyzed site or correct the low-accuracy genotype.

The Imputation method is mainly applied in Genome wide association study (GWAS) or population genetic analysis. LD information is used to amplify the number of data sites on chip, so as to dig up the genotype information related to a specific phenotype to the maximal extent. Alternatively, for low-depth population sequencing data, the haplotype information of the reference population or the analyzed population is used to correct the genotype sites that are erroneously detected due to a too low depth, thereby improving the accuracy of analysis.

In the present disclosure, the principle of correcting low-depth sites in the analyzed sample using haplotype information in the imputation is applied to maternal plasma data, and the LD information is used to comprehensively estimate the fetal nucleic acid concentration of the whole genome (or chromosome level). When performing the imputation on a single sample, the genotype inference algorithms such as IMPUTE2 have the same premise that the analyzed sample is diploid. Therefore, when genotypes that contradict this premise (i.e., more than two haplotypes) are present at some sites, these sites will be regarded as error sites and will be corrected. Since the maternal plasma actually contains information about three types of haplotype information, i.e., two maternal haplotypes and one fetal haplotype inherited from the father, there is a certain probability that the fetal haplotype derived from the father is deemed to be error site and thus corrected during the process of imputation. Such a probability of being corrected is further correlated with the fetal cfDNA concentration in maternal plasma.

Using the information about haplotype and linkage disequilibrium in the genome of the pregnant woman, by means of the application of the haplotype information in imputation, the fetal nucleic acid concentration in a non-invasive prenatal genetic testing can be calculated based on a proportion of sites corrected by the imputation.

When aligning the sequencing data to at least a part of the reference genome, a genomic region with more evident signals can be selected as the alignment region. The mentioned genomic region with more evident signals may be reflected as a region having better coverage (the coverage of the sequencing data on the genome), relatively high minor allele frequency (indicating that the site has a high variation probability in the population), and relatively high proportion of variation sites, for the purposes of further extracting characteristic information and reducing background noise interference, thereby improving the accuracy of fetal nucleic acid concentration estimation.

When selecting a strong linkage region, the strong linkage region can be determined by changing a calculation window size, for example, changing a window of 5mb to a window of 10mb or to a window of the entire chromosome, so as to improve the accuracy by increasing the number of effective sites in each window.

In some embodiments of the present disclosure, the step (3) further includes: step (3-1) of determining a difference ratio between the first genotype information and the second genotype information; and step (3-2) of determining the fetal nucleic acid concentration based on the difference ratio obtained in step (3-1) and a pre-trained formula, where the formula is determined based on a plurality of training samples with known fetal nucleic acid concentrations. In view of a relationship between the plurality of known fetal nucleic acid concentrations and the difference ratio between the first genotype information and the second genotype information, different formulas or models, e.g., the linear regression model, or other models that effectively integrate all information, such as the random forest model or other deep learning models, etc., are used for training to correlate the fetal nucleic acid concentration with the difference ratio. When the difference ratio between the first genotype information and the second genotype information of a nucleic acid sample from certain maternal blood is determined, the fetal nucleic acid concentration in this maternal blood can be determined by means of the pre-trained formula.

When selecting or determining the model, additional and more complete phenotypic information of pregnant women can be added as covariates in the prediction model, thereby optimizing the prediction model and increasing the accuracy of estimation.

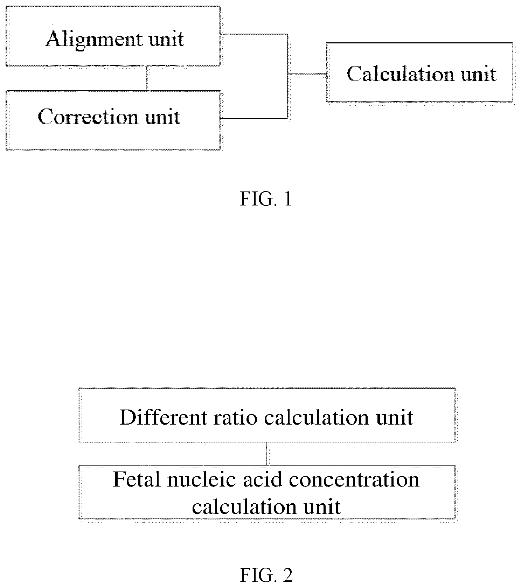

According to another aspect of the present disclosure, the present disclosure provides a device for determining a fetal nucleic acid concentration in maternal blood. The fetal nucleic acid concentration in maternal blood can be determined with such a device, which only requires to use the NIPT data of the maternal plasma without the assistance of other data. In addition, the device can be applied to the sequencing data of ultra-low depth. Besides, the device has not specified the sample type, and both samples involving male fetuses and samples involving female fetuses can be used. As illustrated in FIG. 1, the device includes: an alignment unit configured to determine first genotype information based on alignment between sequencing data and at least a part of a reference genome, where the sequencing data are derived from a nucleic acid sample of the maternal blood; a correction unit connected to the alignment unit, the correction unit being configured to correct the first genotype information based on reference data by using a linkage disequilibrium relationship to obtain second genotype information; and a calculation unit connected to the alignment unit and the correction unit, the calculation unit being configured to determine the fetal nucleic acid concentration based on a difference between the first genotype information and the second genotype information.

In at least some embodiments, the calculation unit, as illustrated in FIG. 2, further includes: a difference ratio calculation unit configured to determine a difference ratio between the first genotype information and the second genotype information; and a fetal nucleic acid concentration calculation unit connected to the difference ratio calculation unit. The fetal nucleic acid concentration calculation unit is configured to determine the fetal nucleic acid concentration based on the difference ratio obtained in the difference ratio calculation unit and a pre-trained formula. The formula is determined based on a plurality of training samples with known fetal nucleic acid concentrations.

The solutions of the present disclosure will be explained below in conjunction with examples. Those skilled in the art will understand that the following exmaples are merely used to illustrate the present disclosure, and should not be regarded as limitations of the scope of the present disclosure. Where specific techniques or conditions are not indicated in the examples, the procedures shall be carried out in accordance with the techniques or conditions described in the literatures in the field or in accordance with the product instructions. The reagents or instruments used without indicating the manufacturers are all conventional products that are commercially available.

**Example 1**

Example 1 provides a method for calculating a fetal nucleic acid concentration in maternal plasma using cfDNA sequencing data of the maternal plasma as input data. The specific steps are as follows:

(1) Preliminary data processing

All the raw sequencing data (in fq format) of samples used for model-training and prediction are aligned to the human reference chromosome hg38 using the samse mode in BWA after quality control. Picard is used to remove duplicated reads in the mapping results and calculate a duplication rate. The mapping results are locally corrected using a base quality value correction BQSR function in the variation detection algorithm such as GATK. A depth distribution of each sample is calculated using the Depth of Coverage function in the variation detection algorithm such as GATK. A population variation detection mode in the variation detection algorithm such as GATK is used to perform the detection of single nucleotide variation (SNV) and small fragment insertion-deletion (Indel).

(2) Extraction of raw genotype information

The results of BWA alignment and Picard duplication-removing (in bam format) in step (1) are taken as input, and the genotype result (in vcf format) based on a depth of the raw reads is output through mpileup function in samtools software. That is, pileup function in the samtools software is used to infer the genotype.

(3) Calculation of proportion of imputation-corrected sites

The whole genome is divided into windows of 5 mb. Each window is for each analyzed sample, and the variation site information and frequency information are used as the population reference data. The variation site information and frequency information may come from existing databases (such as the 1000 genomes, the Hapmap database, and other human population reference genome database), or can also be obtained through calculation by using the population information of the input data itself (i.e., directly calculating the genotype and its corresponding frequency of each locus in the sample to be analyzed). The genotype completion and correction (imputation) is completed by using IMPUTE2 or other genotype imputation algorithms, so as to finally obtain the result of the genotype of each sample (in vcf format). With this obtained genotype and the genotype inferred from the raw reads depth information in (2), a proportion of genotype-inconsistent sites in the two sets of data is calculated as the proportion of imputation-corrected sites. The correction can be performed by referring to the principle of Imputation by Porcu, E.; Sanna, S.; Fuchsberger, C.; Fritsche, L.G., Genotype imputation in genome-wide association studies. Curr Protoc Hum Genet. 2013, Chapter 1, Unit 1.25.

The Imputation is a method of genotype completion and correction for sites with deletion data or low accuracy. Specifically, the linkage disequilibrium (LD) relationship between the analyzed site and its nearby sites with higher accuracy is utilized to find the haplotype that best matches the analyzed site (by using haplotype information in the reference population, or using haplotype information among different individuals of the analyzed population), so as to infer the deletion genotype at the analyzed site or correct the low-accuracy genotype.

The Imputation method is mainly applied in Genome wide association study (GWAS) or population genetic analysis. LD information is used to amplify the number of data sites on chip, so as to dig up the genotype information related to a specific phenotype to the maximal extent. Alternatively, for low-depth population sequencing data, the haplotype information of the reference population or the analyzed population is used to correct the erroneously detected genotype sites due to a too low depth, thereby improving the accuracy of analysis.

In the present disclosure, the principle of correcting low-depth sites in the analyzed sample using haplotype information in the imputation is applied to maternal plasma data, and the LD information is used to comprehensively estimate the fetal nucleic acid concentration of the whole genome (or chromosome level). When performing the imputation on a single sample, the genotype inference algorithms such as IMPUTE2 have the same premise that the analyzed sample is diploid. Therefore, when genotypes that contradict this premise (i.e., more than two haplotypes) exist at some sites, these sites will be regarded as error sites and will be corrected. Since the maternal plasma actually contains information about three types of haplotypes, i.e., two maternal haplotypes and one fetal haplotype inherited from the father, there is a certain probability that the fetal haplotype derived from the father is deemed to be error site and corrected during the process of imputation. Such a probability of being corrected is further correlated with the fetal cfDNA concentration in maternal plasma.

(4) Establishment of fetal nucleic acid concentration prediction model

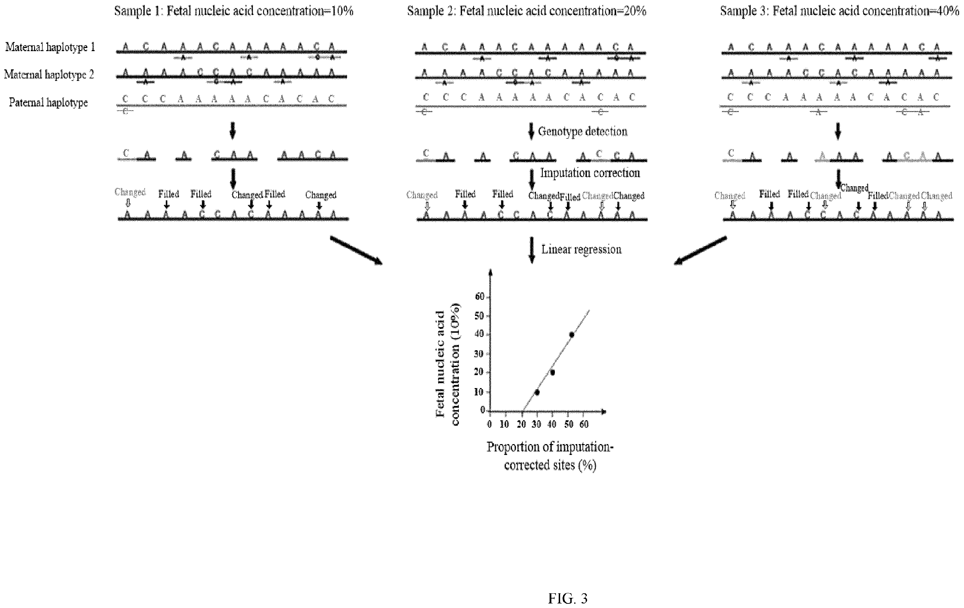

The imputation process for sequencing data is a process of correcting and completing the genotype of the low-accuracy or deletion loci with the aid of high-accuracy loci nearby using the information of linkage disequilibrium between different loci. Currently, IMPUTE2 or other genotype inference algorithms are used to perform imputation on maternal plasma cfDNA data based on the assumption that all sequencing data originate from the same individual, that is, only two haplotypes. Therefore, in the imputation process, the third haplotype composed of paternal cfDNA in the maternal plasma will be regarded as an error site and thus will be corrected. If the sequencing depth is the same (or after the sequencing depth correction), the probability of extracting paternal cfDNA that is different from that of the pregnant woman increases with the increase of the fetal nucleic acid concentration, and thus the proportion of corrected sites also increases (as illustrated in FIG. 3).

As illustrated in FIG. 3, when the paternal cfDNA proportions corresponding to different fetal nucleic acid concentrations increase, the probability that the raw genotype (i.e., the first genotype) is inferred from the paternal cfDNA increases, and thus the probability that these paternal genotypes are corrected to maternal genotypes through the imputation also increases. That is, the difference ratio between the first genotype and the second genotype as mentioned above is increased. Therefore, the fetal free DNA concentration can be inversely inferred by calculating the change in the ratio of the first genotype to the second genotype. As illustrated in FIG. 3, the proportion of imputation-corrected sites varies with different fetal nucleic acid concentrations, indicating the correlation process of indirectly inferring the fetal cfDNA concentration and the genotype change proportion.

Based on the above theory, a linear regression model for predicting the fetal nucleic acid concentration is established by using male fetus maternal plasma cfDNA data of a large sample size (recommended more than 10,000 cases) as the training set, the fetal nucleic acid concentration calculated with Y chromosome depth as the true set (Y value), and the proportion of corrected sites calculated in the above (3) as a covariate (X value), and adding an average sequencing depth, a high-quality sequencing depth, and the duplication rate of the samples as covariates.

The specific formula of the linear regression model is as below:

$$y_i = \beta_0 + \beta_1 x_{i1} + \beta_2 x_{i2} + \ldots + \beta_n x_{in} + \beta_{cov} x_{icov} + \beta_{gcov} x_{igcov} + \beta_{dup} x_{idup} + \varepsilon_i, \ i = 1, \ldots, p,$$

where $y_i$ is a male fetal nucleic acid concentration calculated from a Y chromosome depth corresponding to sample i; $\{x_{t1} \ldots x_{ta}\}$ is a proportion of sites corrected by imputation in each window with respect to all n windows in sample i; $x_{icos}$ is an average sequencing depth corresponding to sample i; $x_{igcos}$ is a sequencing depth of high-quality aligned reads corresponding to sample i; $x_{idup}$ is a duplication rate corresponding to sample i; and p is the total number of samples in the training set.

(5) Prediction of fetal nucleic acid concentration

For all maternal plasma cfDNA samples, the fetal nucleic acid concentration is predicted with the prediction model obtained in the above (4), using the proportion of imputation-corrected sites, the average sequencing depth, the high-quality sequencing depth and the duplication rate of each sample as the covariates.

This method has been preliminarily tested on NIPT ultra-low depth (about 0.1x) SE sequencing data. The data of 10,000 cases with male fetuses as the training set, and the fetal nucleic acid concentration estimated by the Y chromosome depth as the true set are used for the training of linear regression model. Meanwhile, the three variables, i.e., the average sequencing depth, the high-quality sequencing depth and the duplication rate of each sample, are used as covariates of the model to construct the prediction model. Then, the fetal nucleic acid concentrations of 1,000 samples are independently estimated twice with this prediction model, and the correlation between the estimated fetal nucleic acid concentration and the actual fetal nucleic acid concentration (the fetal nucleic acid concentration calculated with the Y chromosome depth) is as follows.

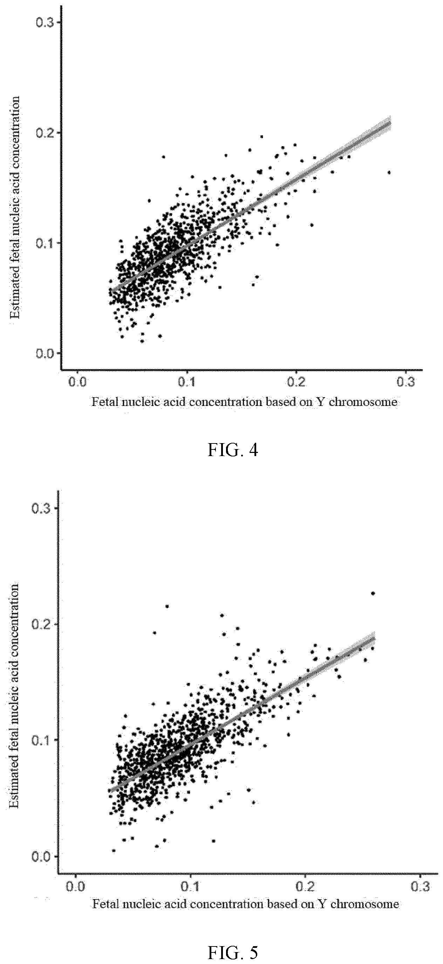

FIG. 4 illustrates the prediction results of the fetal nucleic acid concentrations of a test data set of 1000 samples (test data set 1), which are independently predicted with the model obtained using 10,000 samples as the training set. In the test data set 1, the correlation ($R^2$) between the fetal nucleic acid concentration calculated based on the Y chromosome depth (abscissa) and the fetal nucleic acid concentration calculated by the method of the present disclosure (ordinate) is 0.7318 (95% confidence interval: 0.7016-0.7593).

FIG. 5 illustrates the prediction results of the fetal nucleic acid concentrations of a test data set of 1000 samples (test data set 2), which are independently predicted with the model obtained using 10,000 samples as the training set. In the test data set 2, the correlation ($R^2$) between the fetal nucleic acid concentration calculated based on the Y chromosome depth (abscissa) and the fetal nucleic acid concentration calculated by the method of the present disclosure (ordinate) is 0.7423 (95% confidence interval: 0.7131-0.7689).

Based on the results of Pearson test, the fetal nucleic acid concentration estimated with either of the two test data sets is significantly correlated with the fetal nucleic acid concentration obtained with the Y chromosome depth (p value is less than $2.2 \times 10^{-16}$).

The linear regression correlation results obtained from the training set of 10,000 samples can be referred to the appendix. The values in the appendix are the standard output results of the linear regression model in R. The estimated value (coefficient) is a calculated value of the coefficient corresponding to each input covariate, that is, a parameter of the model obtained from the training set. The parameter can be used to predict a fetal cfDNA concentration of a new sample by being directly brought into the linear model. The standard deviation is an error corresponding to the estimated value. The T value and p value are the significance test results of the corresponding covariates. The significance in the last column is a degree of significance based on the p value. In practical applications, only the more significant covariates (such as p less than 0.05) can be selected for prediction.

In the description of the present disclosure, the terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "plurality of" means at least two, such as two, three, etc., unless otherwise specifically defined.

In the specification, descriptions with reference to the terms "an embodiment", "some embodiments", "examples", "specific examples", or "some examples", etc. mean specific features, structures, materials or characteristics described in conjunction with the embodiment or example are included in at least one embodiment or example of the present disclosure. In this specification, the above terms are illustrative, and do not necessarily refer to the same embodiment or example. Moreover, the described specific features, structures, materials or characteristics can be combined in a suitable manner in any one or more embodiments or examples. In addition, those skilled in the art can combine the different embodiments or examples and the features of the different embodiments or examples described in this specification without contradicting each other.

Although the embodiments of the present disclosure are illustrated and described above, it can be understood that the above-mentioned embodiments are illustrative and should not be construed as limitations of the present disclosure. Those skilled in the art can make changes, modifications, substitutions, and variations based on the above-mentioned embodiments within the scope of the present disclosure.

**Appendix**

Linear model results obtained from the training of 10,000 samples from pregnant women (male fetuses):

| Covariate | Estimated value | Standard deviation | T value | p value | Significance |
|---|---|---|---|---|---|
| Intercept | -7.54E-01 | 8.23E-03 | -91.70 | 2.00E-16 | *** |
| Average sequencing depth | -3.74E-01 | 1.59E-01 | -2.36 | 1.85E-02 | * |
| High-quality sequencing depth | -6.20E-0 1 | 1.75E-01 | -3.55 | 3.89E-04 | *** |
| Duplication rate | 3.13E+00 | 1.74E-01 | 18.02 | 2.00E-16 | *** |

(continued)

| Covariate | | Estimated value | Standard deviation | T value | *p* value | Significance |
|---|---|---|---|---|---|---|
| | chr1.1.5000000 | 5.02E-03 | 1.67E-03 | 3.01 | 2.64E-03 | ** |
| | chr1.5000001.10000000 | 5.01E-03 | 1.68E-03 | 2.97 | 2.95E-03 | ** |
| | chr1.10000001.15000000 | 2.92E-03 | 1.43E-03 | 2.04 | 4.15E-02 | * |
| | chr1.15000001.20000000 | 5.79E-03 | 1.86E-03 | 3.12 | 1.81E-03 | ** |
| | chr1.20000001.25000000 | 4.50E-03 | 1.83E-03 | 2.47 | 1.37E-02 | * |
| | chr1.25000001.30000000 | 1.89E-03 | 1.30E-03 | 1.46 | 1.45E-01 | |
| | chr1.30000001.35000000 | 8.88E-03 | 2.15E-03 | 4.13 | 3.69E-05 | *** |
| | chr1.35000001.40000000 | 3.27E-03 | 1.62E-03 | 2.01 | 4.41E-02 | * |
| | chr1.40000001.45000000 | 6.21E-03 | 1.69E-03 | 3.68 | 2.33E-04 | *** |
| Proportion of genotype sites changed by Imputation (5Mb window) | chr1.45000001.50000000 | 2.03E-03 | 1.46E-03 | 1.39 | 1.64E-01 | |
| | chr1.50000001.55000000 | 2.57E-03 | 1.19E-03 | 2.15 | 3.14E-02 | * |
| | chr1.55000001.60000000 | 7.52E-03 | 2.28E-03 | 3.29 | 9.97E-04 | *** |
| | chr1.60000001.65000000 | 1.11E-03 | 1.81E-03 | 0.61 | 5.40E-01 | |
| | chr1.65000001.70000000 | 4.92E-03 | 1.83E-03 | 2.69 | 7.07E-03 | ** |
| | chr1.70000001.75000000 | 3.04E-03 | 1.67E-03 | 1.82 | 6.88E-02 | |
| | chr1.75000001.80000000 | 4.29E-03 | 1.81E-03 | 2.37 | 1.79E-02 | * |
| | chr1.80000001.85000000 | 4.38E-03 | 1.87E-03 | 2.34 | 1.93E-02 | * |
| | chr1.85000001.90000000 | 4.77E-03 | 1.97E-03 | 2.42 | 1.55E-02 | * |
| | chr1.90000001.95000000 | 1.85E-03 | 1.70E-03 | 1.09 | 2.76E-01 | |
| | chr1.95000001.100000000 | 5.93E-03 | 1.90E-03 | 3.12 | 1.83E-03 | ** |

| | | | | | |
|---|---|---|---|---|---|
| chr1. 10000000 1. 105000000 | 2.48E-03 | 1.92E-03 | 1.29 | 1.97E-01 | |
| chr1.105000001.110000000 | 4.59E-03 | 1.94E-03 | 2.37 | 1.76E-02 | * |
| chr1.110000001.115000000 | 4.16E-03 | 1.80E-03 | 2.31 | 2.10E-02 | * |
| chr1.115000001.120000000 | 1.94E-03 | 1.88E-03 | 1.03 | 3.02E-01 | |
| chr1.120000001.125000000 | 1.15E-03 | 7.02E-04 | 1.64 | 1.01E-01 | |
| chr1.140000001.145000000 | 2.92E-03 | 1.16E-03 | 2.51 | 1.21E-02 | * |
| chr1.145000001.150000000 | 1.59E-03 | 9.89E-04 | 1.61 | 1.08E-01 | |
| chr1.150000001.155000000 | 2.91E-03 | 1.52E-03 | 1.92 | 5.55E-02 | |
| chr1.155000001.160000000 | 1.31E-03 | 1.40E-03 | 0.94 | 3.50E-01 | |
| chr1.160000001.165000000 | 5.77E-03 | 1.84E-03 | 3.14 | 1.69E-03 | ** |
| chr1.165000001.170000000 | 3.05E-03 | 1.80E-03 | 1.70 | 8.97E-02 | |
| chr1.170000001.175000000 | 1.35E-03 | 1.82E-03 | 0.74 | 4.58E-01 | |
| chr1.175000001.180000000 | 2.65E-03 | 1.64E-03 | 1.62 | 1.06E-01 | |
| chr1.180000001.185000000 | 7.05E-03 | 1.57E-03 | 4.49 | 7.21E-06 | *** |
| chr1.185000001.190000000 | 2.41E-03 | 1.70E-03 | 1.41 | 1.57E-01 | |
| chr1.190000001.195000000 | 2.64E-03 | 1.99E-03 | 1.33 | 1.85E-01 | |
| chr1.195000001.200000000 | 5.04E-03 | 1.80E-03 | 2.80 | 5.17E-03 | ** |
| chr1.200000001.205000000 | 4.19E-03 | 1.99E-03 | 2.10 | 3.55E-02 | * |
| chr1.205000001.210000000 | 2.63E-03 | 1.69E-03 | 1.56 | 1.20E-01 | |
| chr1.210000001.215000000 | 2.02E-03 | 1.83E-03 | 1.11 | 2.68E-01 | |
| chr1.215000001.220000000 | 5.44E-03 | 1.98E-03 | 2.76 | 5.88E-03 | ** |
| chr1.220000001.225000000 | 5.52E-03 | 2.02E-03 | 2.74 | 6.19E-03 | ** |
| chr1.225000001.230000000 | 1.91E-03 | 1.54E-03 | 1.24 | 2.14E-01 | |
| chr1.230000001.235000000 | 9.08E-03 | 1.96E-03 | 4.64 | 3.48E-06 | *** |
| chr1.235000001.240000000 | 2.33E-03 | 1.77E-03 | 1.32 | 1.87E-01 | |
| chr 1.240000001.245000000 | 6.15E-03 | 2.00E-03 | 3.07 | 2.15E-03 | ** |
| chr1.245000001.250000000 | 3.55E-03 | 1.66E-03 | 2.14 | 3.21E-02 | * |
| chr10.1.5000000 | 5.86E-03 | 2.22E-03 | 2.63 | 8.45E-03 | ** |
| chr10.5000001.10000000 | 6.62E-03 | 2.20E-03 | 3.01 | 2.59E-03 | ** |
| chr10.10000001.15000000 | 4.55E-03 | 1.99E-03 | 2.28 | 2.24E-02 | * |
| chr10.15000001.20000000 | 7.02E-03 | 2.27E-03 | 3.10 | 1.95E-03 | ** |
| chr10.20000001.25000000 | 4.68E-03 | 1.70E-03 | 2.75 | 5.89E-03 | ** |
| chr10.25000001.30000000 | 8.37E-03 | 2.23E-03 | 3.76 | 1.71E-04 | *** |
| chr10.30000001.35000000 | 1.97E-03 | 1.73E-03 | 1.14 | 2.55E-01 | |
| chr 10.35000001.40000000 | 1.40E-03 | 1.30E-03 | 1.08 | 2.82E-01 | |

| | | | | | |
|---|---|---|---|---|---|
| chr10.40000001.45000000 | 3.98E-03 | 1.57E-03 | 2.54 | 1.11E-02 | * |
| chr10.45000001.50000000 | 2.49E-03 | 1.47E-03 | 1.70 | 8.98E-02 | |
| chr10.50000001.55000000 | 8.08E-03 | 2.08E-03 | 3.89 | 1.02E-04 | *** |
| chr10.55000001.60000000 | 5.20E-03 | 1.75E-03 | 2.96 | 3.05E-03 | ** |
| chr10.60000001.65000000 | 4.09E-03 | 1.82E-03 | 2.24 | 2.48E-02 | * |
| chr10.65000001.70000000 | 8.72E-03 | 2.23E-03 | 3.91 | 9.35E-05 | *** |
| chr10.70000001.75000000 | 4.91E-03 | 1.63E-03 | 3.02 | 2.52E-03 | ** |
| chr10.75000001.80000000 | 3 .98E-04 | 1.54E-03 | 0.26 | 7.95E-01 | |
| chr10.80000001.85000000 | 5.53E-03 | 1.82E-03 | 3.05 | 2.32E-03 | ** |
| chr10.85000001.90000000 | 6.73E-03 | 1.86E-03 | 3.63 | 2.88E-04 | *** |
| chr10.90000001.95000000 | 5.61E-03 | 1.68E-03 | 3.34 | 8.31E-04 | *** |
| chr10.95000001.100000000 | 2.70E-03 | 1.78E-03 | 1.51 | 1.30E-01 | |
| chr10.100000001.105000000 | 6.30E-03 | 1.56E-03 | 4.04 | 5.37E-05 | *** |
| chr10.105000001.110000000 | 5.86E-03 | 1.75E-03 | 3.34 | 8.36E-04 | *** |
| chr10.110000001.115000000 | 5.34E-03 | 1.84E-03 | 2.90 | 3.78E-03 | ** |
| chr10.115000001.120000000 | 7.68E-03 | 1.80E-03 | 4.26 | 2.07E-05 | *** |
| chr10.120000001.125000000 | 7.16E-04 | 2.18E-03 | 0.33 | 7.43E-01 | |
| chr10.125000001.130000000 | 4.62E-03 | 2.21E-03 | 2.09 | 3.66E-02 | * |
| chr10.130000001.135000000 | 9.08E-03 | 1.97E-03 | 4.60 | 4.20E-06 | *** |
| chr10.135000001.140000000 | 3.54E-04 | 6.53E-04 | 0.54 | 5.88E-01 | |
| chr11.1.5000000 | 5.54E-03 | 1.79E-03 | 3.10 | 1.93E-03 | ** |
| chr11.5000001.10000000 | 4.92E-03 | 2.1 lE-03 | 2.33 | 2.01E-02 | * |
| chr11.10000001.15000000 | 5.17E-03 | 2.10E-03 | 2.46 | 1.39E-02 | * |
| chr11.15000001.20000000 | 2.98E-03 | 2.09E-03 | 1.42 | 1.55E-01 | |
| chr11.20000001.25000000 | 8.55E-03 | 2.38E-03 | 3.59 | 3.36E-04 | *** |
| chr11.25000001.30000000 | 8.04E-04 | 1.81E-03 | 0.44 | 6.57E-01 | |
| chr11.30000001.35000000 | 1.96E-03 | 1.81E-03 | 1.08 | 2.78E-01 | |
| chr11.35000001.40000000 | 2.94E-03 | 1.71E-03 | 1.72 | 8.47E-02 | |
| chr11.40000001.45000000 | 6.83E-03 | 1.95E-03 | 3.51 | 4.44E-04 | *** |
| chr11.45000001.50000000 | 1.37E-03 | 1.32E-03 | 1.04 | 3.00E-01 | |
| chr11.50000001.55000000 | 6.41E-05 | 7.33E-04 | 0.09 | 9.30E-01 | |
| chr11.55000001.60000000 | 4.50E-05 | 1.61E-03 | 0.03 | 9.78E-01 | |
| chr11.60000001.65000000 | 1.87E-03 | 1.48E-03 | 1.27 | 2.06E-01 | |
| chr11.65000001.70000000 | 3.78E-03 | 1.52E-03 | 2.49 | 1.27E-02 | * |
| chr11.70000001.75000000 | 4.72E-03 | 1.61E-03 | 2.94 | 3.30E-03 | ** |

| | | | | | |
|---|---|---|---|---|---|
| chr11.75000001.80000000 | 3.34E-03 | 1.55E-03 | 2.16 | 3.07E-02 | * |
| chr11.80000001.85000000 | 6.98E-03 | 1.75E-03 | 3.98 | 6.97E-05 | *** |
| chr11.85000001.90000000 | 3.25E-03 | 1.92E-03 | 1.70 | 9.02E-02 | |
| chr11.90000001.95000000 | 9.87E-03 | 1.98E-03 | 4.98 | 6.53E-07 | *** |
| chr11.95000001.100000000 | 6.33E-03 | 2.08E-03 | 3.04 | 2.37E-03 | ** |
| chr11.100000001.105000000 | 4.72E-03 | 2.15E-03 | 2.19 | 2.84E-02 | * |
| chr11.105000001.110000000 | 3.13E-03 | 1.86E-03 | 1.68 | 9.27E-02 | |
| chr11.110000001.115000000 | 6.99E-03 | 2.19E-03 | 3.20 | 1.40E-03 | ** |
| chr11.115000001.120000000 | 6.53E-03 | 1.93E-03 | 3.39 | 7.06E-04 | *** |
| chr11.120000001.125000000 | 3.33E-03 | 1.64E-03 | 2.04 | 4.19E-02 | * |
| chr11.125000001.130000000 | 5.39E-03 | 1.85E-03 | 2.92 | 3.56E-03 | ** |
| chr11.130000001.135000000 | 5.77E-03 | 1.95E-03 | 2.95 | 3.16E-03 | ** |
| chr12.1.5000000 | 1.36E-04 | 1.74E-03 | 0.08 | 9.38E-01 | |
| chr12.5000001.10000000 | 4.91E-03 | 1.56E-03 | 3.15 | 1.62E-03 | ** |
| chr12.10000001.15000000 | 7.96E-03 | 1.74E-03 | 4.57 | 4.91E-06 | *** |
| chr12.15000001.20000000 | 3.10E-03 | 1.94E-03 | 1.60 | 1.10E-01 | |
| chr12.20000001.25000000 | 5.03E-03 | 1.58E-03 | 3.19 | 1.45E-03 | ** |
| chr12.25000001.30000000 | 7.96E-03 | 2.03E-03 | 3.93 | 8.62E-05 | *** |
| chr12.30000001.35000000 | 8.51E-03 | 1.80E-03 | 4.73 | 2.28E-06 | *** |
| chr12.35000001.40000000 | 4.54E-04 | 9.64E-04 | 0.47 | 6.38E-01 | |
| chr12.40000001.45000000 | 3.68E-03 | 2.02E-03 | 1.82 | 6.89E-02 | |
| chr12.45000001.50000000 | 4.69E-03 | 1.89E-03 | 2.48 | 1.31E-02 | * |
| chr12.50000001.55000000 | 3.56E-03 | 1.65E-03 | 2.16 | 3.1 lE-02 | * |
| chr12.55000001.60000000 | 2.32E-03 | 1.48E-03 | 1.57 | 1.16E-01 | |
| chr12.60000001.65000000 | 4.74E-03 | 1.80E-03 | 2.64 | 8.36E-03 | ** |
| chr12.65000001.70000000 | 3.91E-03 | 1.83E-03 | 2.14 | 3.25E-02 | * |
| chr12.70000001.75000000 | 5.53E-03 | 1.77E-03 | 3.13 | 1.77E-03 | ** |
| chr12.75000001.80000000 | 5.71E-03 | 1.84E-03 | 3.10 | 1.93E-03 | ** |
| chr12.80000001.85000000 | 3.89E-03 | 2.12E-03 | 1.84 | 6.61E-02 | |
| chr12.85000001.90000000 | 3.83E-03 | 1.37E-03 | 2.80 | 5.16E-03 | ** |
| chr12.90000001.95000000 | 7.21E-03 | 1.86E-03 | 3.87 | 1. 10E-04 | *** |
| chr12.95000001.100000000 | 7.35E-03 | 2.04E-03 | 3.60 | 3.22E-04 | *** |
| chr12.100000001.105000000 | 5.89E-03 | 1.83E-03 | 3.22 | 1.30E-03 | ** |
| chr12.105000001.110000000 | 7.91E-03 | 2.26E-03 | 3.51 | 4.58E-04 | *** |
| chr12.110000001.115000000 | 4.42E-03 | 1.47E-03 | 3.00 | 2.71E-03 | ** |

| | | | | | |
|---|---|---|---|---|---|
| chr12.115000001.120000000 | 5.05E-03 | 1.77E-03 | 2.86 | 4.25E-03 | ** |
| chr12.120000001.125000000 | 2.72E-03 | 1.66E-03 | 1.64 | 1.01E-01 | |
| chr12.125000001.130000000 | 4.91E-03 | 2.11E-03 | 2.33 | 1.98E-02 | * |
| chr12.130000001.135000000 | 2.03E-03 | 1.78E-03 | 1.14 | 2.53E-01 | |
| chr13.15000001.20000000 | 1.77E-03 | 7.66E-04 | 2.31 | 2.08E-02 | * |
| chr13.20000001.25000000 | 1.93E-03 | 1.96E-03 | 0.98 | 3.25E-01 | |
| chr13.25000001.30000000 | 6.00E-03 | 2.12E-03 | 2.83 | 4.74E-03 | ** |
| chr13.30000001.35000000 | 3.86E-03 | 2.05E-03 | 1.88 | 6.04E-02 | |
| chr13.35000001.40000000 | 6.19E-03 | 1.76E-03 | 3.51 | 4.45E-04 | *** |
| chr13.40000001.45000000 | 5.25E-03 | 2.14E-03 | 2.46 | 1.41E-02 | * |
| chr13.45000001.50000000 | 5.49E-03 | 1.72E-03 | 3.18 | 1.46E-03 | ** |
| chr13.50000001.55000000 | 2.22E-03 | 1.61E-03 | 1.38 | 1.67E-01 | |
| chr13.55000001.60000000 | 3.72E-04 | 1.71E-03 | 0.22 | 8.28E-01 | |
| chr13.60000001.65000000 | 5.22E-03 | 1.52E-03 | 3.44 | 5.83E-04 | *** |
| chr13.65000001.70000000 | 7.52E-03 | 1.98E-03 | 3.80 | 1.47E-04 | *** |
| chr13.70000001.75000000 | 5.33E-03 | 2.00E-03 | 2.67 | 7.57E-03 | ** |
| chr13.75000001.80000000 | 3.44E-03 | 1.76E-03 | 1.95 | 5.11E-02 | |
| chr13.80000001.85000000 | 6.18E-03 | 1.91E-03 | 3.24 | 1.20E-03 | ** |
| chr13.85000001.90000000 | 2.76E-03 | 1.87E-03 | 1.48 | 1.40E-01 | |
| chr13.90000001.95000000 | 7.38E-03 | 2.17E-03 | 3.40 | 6.77E-04 | *** |
| chr13.95000001.100000000 | 6.44E-03 | 1.88E-03 | 3.43 | 6.04E-04 | *** |
| chr13.100000001.105000000 | 3.79E-03 | 2.14E-03 | 1.77 | 7.67E-02 | |
| chr13.105000001.110000000 | 6.13E-03 | 2.10E-03 | 2.92 | 3.57E-03 | ** |
| chr13.110000001.115000000 | 4.88E-03 | 1.97E-03 | 2.48 | 1.31E-02 | * |
| chr13.115000001.120000000 | -7.1 IE-04 | 7.62E-04 | -0.93 | 3.51E-01 | |
| chr14.15000001.20000000 | -2.20E-03 | 1.86E-03 | -1.19 | 2.35E-01 | |
| chr14.20000001.25000000 | 4.89E-03 | 1.93E-03 | 2.54 | 1.12E-02 | * |
| chr14.25000001.30000000 | 7.32E-03 | 1.85E-03 | 3.95 | 7.88E-05 | *** |
| chr14.30000001.35000000 | 4.06E-03 | 1.42E-03 | 2.86 | 4.28E-03 | ** |
| chr14.35000001.40000000 | 3.74E-03 | 1.91E-03 | 1.97 | 4.94E-02 | * |
| chr14.40000001.45000000 | 6.82E-03 | 2.19E-03 | 3.11 | 1.85E-03 | ** |
| chr14.45000001.50000000 | 1.93E-03 | 1.76E-03 | 1.10 | 2.73E-01 | |
| chr14.50000001.55000000 | 1.38E-03 | 1.86E-03 | 0.74 | 4.58E-01 | |
| chr14.55000001.60000000 | 4.00E-03 | 1.87E-03 | 2.14 | 3.21E-02 | * |
| chr14.60000001.65000000 | 1.32E-03 | 1.94E-03 | 0.68 | 4.95E-01 | |

| | | | | | |
|---|---|---|---|---|---|
| chr14.65000001.70000000 | 2.30E-03 | 9.32E-04 | 2.47 | 1.36E-02 | * |
| chr14.70000001.75000000 | 6.41E-03 | 1.88E-03 | 3.41 | 6.53E-04 | *** |
| chr14.75000001.80000000 | 1.00E-03 | 1.86E-03 | 0.54 | 5.90E-01 | |
| chr14.80000001.85000000 | 3.41E-03 | 2.06E-03 | 1.66 | 9.76E-02 | |
| chr14.85000001.90000000 | -1.14E-03 | 1.94E-03 | -0.59 | 5.55E-01 | |
| chr14.90000001.95000000 | 6.69E-03 | 1.89E-03 | 3.54 | 3.98E-04 | *** |
| chr14.95000001.100000000 | 7.98E-03 | 2.10E-03 | 3.80 | 1.43E-04 | *** |
| chr14.100000001.105000000 | 5.43E-03 | 1.70E-03 | 3.20 | 1.40E-03 | ** |
| chr14.105000001.110000000 | 1.50E-03 | 1.23E-03 | 1.21 | 2.25E-01 | |
| chr15.15000001.20000000 | NA | NA | NA | NA | |
| chr15.20000001.25000000 | 2.53E-03 | 1.21E-03 | 2.09 | 3.70E-02 | * |
| chr15.25000001.30000000 | 1.23E-03 | 1.66E-03 | 0.74 | 4.58E-01 | |
| chr15.30000001.35000000 | 3.47E-03 | 1.67E-03 | 2.07 | 3.84E-02 | * |
| chr15.35000001.40000000 | 7.41E-03 | 1.89E-03 | 3.93 | 8.69E-05 | *** |
| chr15.40000001.45000000 | -3.36E-04 | 1.04E-03 | -0.32 | 7.47E-01 | |
| chr15.45000001.50000000 | 6.54E-04 | 1.79E-03 | 0.37 | 7.15E-01 | |
| chr15.50000001.55000000 | 4.13E-03 | 1.92E-03 | 2.16 | 3.12E-02 | * |
| chr15.55000001.60000000 | -8.54E-04 | 1.93E-03 | -0.44 | 6.58E-01 | |
| chr15.60000001.65000000 | 1.32E-03 | 1.74E-03 | 0.76 | 4.48E-01 | |
| chr15.65000001.70000000 | 5.86E-03 | 1.54E-03 | 3.82 | 1.34E-04 | *** |
| chr15.70000001.75000000 | 3.85E-03 | 1.66E-03 | 2.32 | 2.04E-02 | * |
| chr15.75000001.80000000 | 3.57E-04 | 1.68E-03 | 0.21 | 8.32E-01 | |
| chr15.80000001.85000000 | 2.24E-03 | 1.60E-03 | 1.40 | 1.62E-01 | |
| chr15.85000001.90000000 | 4.59E-03 | 1.92E-03 | 2.39 | 1.67E-02 | * |
| chr15.90000001.95000000 | 3.22E-03 | 2.09E-03 | 1.54 | 1.24E-01 | |
| chr15.95000001.100000000 | 7.17E-03 | 2.03E-03 | 3.54 | 4.03E-04 | *** |
| chr15.100000001.105000000 | 2.85E-03 | 1.35E-03 | 2.11 | 3.53E-02 | * |
| chr16.1.5000000 | 1.45E-03 | 1.37E-03 | 1.06 | 2.88E-01 | |
| chr16.5000001.10000000 | 3.67E-03 | 1.71E-03 | 2.15 | 3.19E-02 | * |
| chr16.10000001.15000000 | -2.33E-04 | 1.99E-03 | -0.12 | 9.07E-01 | |
| chr16.15000001.20000000 | 5.25E-04 | 1.57E-03 | 0.34 | 7.37E-01 | |
| chr16.20000001.25000000 | 4.18E-03 | 1.53E-03 | 2.74 | 6.16E-03 | ** |
| chr16.25000001.30000000 | 2.12E-03 | 1.38E-03 | 1.54 | 1.24E-01 | |
| chr16.30000001.35000000 | 2.08E-03 | 9.24E-04 | 2.25 | 2.43E-02 | * |
| chr16.35000001.40000000 | 1.42E-03 | 1.25E-03 | 1.14 | 2.55E-01 | |

| | | | | | |
|---|---|---|---|---|---|
| chr16.45000001.50000000 | 3.86E-03 | 1.02E-03 | 3.81 | 1.43E-04 | *** |
| chr16.50000001.55000000 | 5.06E-03 | 1.68E-03 | 3.01 | 2.61E-03 | ** |
| chr16.55000001.60000000 | 5.44E-03 | 2.24E-03 | 2.43 | 1.52E-02 | * |
| chr16.60000001.65000000 | 4.81E-03 | 2.12E-03 | 2.26 | 2.36E-02 | * |
| chr16.65000001.70000000 | 6.22E-04 | 1.37E-03 | 0.45 | 6.51E-01 | |
| chr16.70000001.75000000 | 6.58E-04 | 1.51E-03 | 0.44 | 6.62E-01 | |
| chr16.75000001.80000000 | 2.53E-03 | 2.23E-03 | 1.14 | 2.56E-01 | |
| chr16.80000001.85000000 | 8.61E-03 | 2.31E-03 | 3.72 | 2.01E-04 | *** |
| chr116.85000001.90000000 | 5.99E-03 | 1.98E-03 | 3.03 | 2.46E-03 | ** |
| chr16.90000001.95000000 | 1.18E-03 | 7.34E-04 | 1.60 | 1.09E-01 | |
| chr17.1.5000000 | 2.81E-03 | 1.32E-03 | 2.14 | 3.27E-02 | * |
| chr17.5000001.10000000 | 4.40E-03 | 1.73E-03 | 2.54 | 1.11E-02 | * |
| chr17.10000001.15000000 | 8.25E-03 | 2.12E-03 | 3.90 | 9.67E-05 | *** |
| chr17.15000001.20000000 | 4.41E-03 | 1.53E-03 | 2.88 | 3.98E-03 | ** |
| chr17.20000001.25000000 | 7.11E-04 | 8.21E-04 | 0.87 | 3.86E-01 | |
| chr17.25000001.30000000 | 3.73E-03 | 1.33E-03 | 2.81 | 4.94E-03 | ** |
| chr17.30000001.35000000 | 6.86E-03 | 2.06E-03 | 3.33 | 8.83E-04 | *** |
| chr17.35000001.40000000 | 4.20E-04 | 1.59E-03 | 0.27 | 7.91E-01 | |
| chr17.40000001.45000000 | 3.12E-03 | 1.20E-03 | 2.60 | 9.22E-03 | ** |
| chr17.45000001.50000000 | 5.61E-03 | 1.75E-03 | 3.21 | 1.34E-03 | ** |
| chr17.50000001.55000000 | 2.36E-03 | 1.76E-03 | 1.34 | 1.81E-01 | |
| chr17.55000001.60000000 | 9.93E-04 | 1.09E-03 | 0.91 | 3.61E-01 | |
| chr17.60000001.65000000 | 3.96E-03 | 1.27E-03 | 3.12 | 1.82E-03 | ** |
| chr17.65000001.70000000 | 3.43E-03 | 1.77E-03 | 1.94 | 5.30E-02 | |
| chr17.70000001.75000000 | 5.74E-03 | 1.56E-03 | 3.67 | 2.44E-04 | *** |
| chr17.75000001.80000000 | 5.97E-03 | 1.63E-03 | 3.67 | 2.46E-04 | *** |
| chr17.80000001.85000000 | 2.61E-04 | 8.99E-04 | 0.29 | 7.71E-01 | |
| chr18.1.5000000 | 6.78E-03 | 2.18E-03 | 3.11 | 1.87E-03 | ** |
| chr18.5000001.10000000 | 6.66E-03 | 1.91E-03 | 3.49 | 4.91E-04 | *** |
| chr18.10000001.15000000 | 1.36E-03 | 1.92E-03 | 0.71 | 4.80E-01 | |
| chr18.15000001.20000000 | 9.21E-04 | 7.91E-04 | 1.17 | 2.44E-01 | |
| chr18.20000001.25000000 | 4.15E-03 | 1.82E-03 | 2.28 | 2.27E-02 | * |
| chr18.25000001.30000000 | 7.12E-03 | 2.14E-03 | 3.32 | 8.98E-04 | *** |
| chr18.30000001.35000000 | 1.49E-03 | 1.51E-03 | 0.99 | 3.23E-01 | |
| chr18.35000001.40000000 | 2.26E-03 | 2.04E-03 | 1.10 | 2.70E-01 | |

| | | | | | |
|---|---|---|---|---|---|
| chr18.40000001.45000000 | 3.19E-03 | 2.13E-03 | 1.50 | 1.35E-01 | |
| chr18.45000001.50000000 | 2.66E-03 | 1.96E-03 | 1.36 | 1.74E-01 | |
| chr18.50000001.55000000 | 5.27E-03 | 1.75E-03 | 3.02 | 2.52E-03 | ** |
| chr18.55000001.60000000 | 4.63E-03 | 1.77E-03 | 2.62 | 8.80E-03 | ** |
| chr18.60000001.65000000 | 5.23E-03 | 2.05E-03 | 2.55 | 1.09E-02 | * |
| chr18.65000001.70000000 | 9.81E-03 | 2.14E-03 | 4.58 | 4.62E-06 | *** |
| chr18.70000001.75000000 | 6.97E-03 | 1.99E-03 | 3.50 | 4.60E-04 | *** |
| chr18.75000001.80000000 | 3.00E-03 | 1.85E-03 | 1.63 | 1.04E-01 | |
| chr19.1.5000000 | 7.62E-04 | 1.15E-03 | 0.66 | 5.08E-01 | |
| chr19.5000001.10000000 | 1.66E-03 | 1.31E-03 | 1.26 | 2.07E-01 | |
| chr19.10000001.15000000 | 1.39E-03 | 1.18E-03 | 1.17 | 2.41E-01 | |
| chr19.15000001.20000000 | 4.17E-04 | 1.62E-03 | 0.26 | 7.97E-01 | |
| chr19.20000001.25000000 | 1.62E-03 | 1.85E-03 | 0.88 | 3.79E-01 | |
| chr19.25000001.30000000 | 4.56E-03 | 1.49E-03 | 3.07 | 2.15E-03 | ** |
| chr19.30000001.35000000 | 3.81E-03 | 1.70E-03 | 2.24 | 2.54E-02 | * |
| chr119.35000001.40000000 | 2.83E-03 | 1.55E-03 | 1.82 | 6.81E-02 | |
| chr19.40000001.45000000 | 2.83E-03 | 1.64E-03 | 1.73 | 8.45E-02 | |
| chr19.45000001.50000000 | 2.13E-03 | 1.26E-03 | 1.69 | 9.06E-02 | |
| chr19.50000001.55000000 | 5.36E-03 | 1.38E-03 | 3.88 | 1.04E-04 | *** |
| chr19.55000001.60000000 | 3.63E-03 | 1.35E-03 | 2.68 | 7.28E-03 | ** |
| chr2.1.5000000 | 7.31E-03 | 2.26E-03 | 3.24 | 1.21E-03 | ** |
| chr2.5000001.10000000 | 3.53E-03 | 1.88E-03 | 1.88 | 6.01E-02 | |
| chr2.10000001.15000000 | 5.85E-03 | 1.95E-03 | 3.00 | 2.73E-03 | ** |
| chr2.15000001.20000000 | 5.11E-03 | 1.87E-03 | 2.73 | 6.38E-03 | ** |
| chr2.20000001.25000000 | 1.71E-03 | 1.57E-03 | 1.09 | 2.76E-01 | |
| chr2.25000001.30000000 | 1.57E-03 | 1.45E-03 | 1.08 | 2.81E-01 | |
| chr2.30000001.35000000 | 5.72E-03 | 2.03E-03 | 2.82 | 4.77E-03 | ** |
| chr2.35000001.40000000 | 3.42E-03 | 1.88E-03 | 1.82 | 6.92E-02 | |
| chr2.40000001.45000000 | 5.00E-03 | 1.97E-03 | 2.54 | 1.11E-02 | * |
| chr2.45000001.50000000 | 3.05E-03 | 1.98E-03 | 1.54 | 1.23E-01 | |
| chr2.50000001.55000000 | 3.20E-03 | 2.38E-03 | 1.35 | 1.78E-01 | |
| chr2.55000001.60000000 | 4.58E-03 | 1.84E-03 | 2.49 | 1.29E-02 | * |
| chr2.60000001.65000000 | 2.01E-03 | 1.42E-03 | 1.41 | 1.59E-01 | |
| chr2.65000001.70000000 | 3.93E-03 | 2.15E-03 | 1.83 | 6.77E-02 | |
| chr2.70000001.75000000 | 1.57E-03 | 1.80E-03 | 0.87 | 3.82E-01 | |

| | | | | | |
|---|---|---|---|---|---|
| chr2.75000001.80000000 | 3.55E-03 | 2.30E-03 | 1.54 | 1.23E-01 | |
| chr2.80000001.85000000 | 2.60E-03 | 1.37E-03 | 1.91 | 5.65E-02 | |
| chr2.85000001.90000000 | 1.46E-03 | 1.22E-03 | 1.19 | 2.33E-01 | |
| chr2.90000001.95000000 | -9.40E-04 | 8.89E-04 | -1.06 | 2.90E-01 | |
| chr2.95000001.100000000 | 1.38E-03 | 1.17E-03 | 1.18 | 2.40E-01 | |
| chr2.100000001.105000000 | 5.89E-03 | 1.77E-03 | 3.32 | 8.95E-04 | *** |
| chr2.105000001.110000000 | 4.52E-03 | 1.76E-03 | 2.57 | 1.03E-02 | * |
| chr2.110000001.115000000 | 2.56E-03 | 1.64E-03 | 1.56 | 1.18E-01 | |
| chr2.115000001.120000000 | 1.70E-03 | 1.89E-03 | 0.90 | 3.66E-01 | |
| chr2.120000001.125000000 | 3.10E-03 | 1.88E-03 | 1.65 | 9.95E-02 | |
| chr2.125000001.130000000 | 2.90E-03 | 1.74E-03 | 1.67 | 9.59E-02 | |
| chr2.130000001.135000000 | 8.91E-03 | 1.83E-03 | 4.88 | 1.09E-06 | *** |
| chr2.135000001.140000000 | 2.73E-03 | 1.94E-03 | 1.41 | 1.59E-01 | |
| chr2.140000001.145000000 | 5.04E-03 | 2.16E-03 | 2.33 | 1.98E-02 | * |
| chr2.145000001.150000000 | 1.91E-03 | 1.51E-03 | 1.27 | 2.04E-01 | |
| chr2.150000001.155000000 | 5.86E-03 | 1.89E-03 | 3.11 | 1.88E-03 | ** |
| chr2.155000001.160000000 | 2.39E-03 | 1.38E-03 | 1.74 | 8.21E-02 | |
| chr2.160000001.165000000 | 7.30E-03 | 1.86E-03 | 3.93 | 8.52E-05 | *** |
| chr2.165000001.170000000 | 5.52E-03 | 2.17E-03 | 2.54 | 1.10E-02 | * |
| chr2.170000001.175000000 | 5.94E-04 | 1.76E-03 | 0.34 | 7.35E-01 | |
| chr2.175000001.180000000 | 3.65E-03 | 1.43E-03 | 2.55 | 1.07E-02 | * |
| chr2.180000001.185000000 | 7.15E-03 | 2.12E-03 | 3.38 | 7.36E-04 | *** |
| chr2.185000001.190000000 | -8.89E-04 | 1.18E-03 | -0.76 | 4.50E-01 | |
| chr2.190000001.195000000 | 3.75E-03 | 1.49E-03 | 2.52 | 1.18E-02 | * |
| chr2.195000001.200000000 | 4.01E-03 | 1.37E-03 | 2.92 | 3.55E-03 | ** |
| chr2.200000001.205000000 | 3.70E-03 | 1.71E-03 | 2.17 | 3.00E-02 | * |
| chr2.205000001.210000000 | 4.24E-03 | 1.71E-03 | 2.48 | 1.32E-02 | * |
| chr2.210000001.215000000 | 4.64E-03 | 1.60E-03 | 2.90 | 3.78E-03 | ** |
| chr2.215000001.220000000 | 5.56E-03 | 1.77E-03 | 3.14 | 1.69E-03 | ** |
| chr2.220000001.225000000 | 6.00E-03 | 2.16E-03 | 2.78 | 5.50E-03 | ** |
| chr2.225000001.230000000 | 4.43E-03 | 2.05E-03 | 2.16 | 3.1 IE-02 | * |
| chr2.230000001.235000000 | 5.87E-03 | 2.03E-03 | 2.89 | 3.86E-03 | ** |
| chr2.235000001.240000000 | 4.81E-03 | 1.85E-03 | 2.60 | 9.27E-03 | ** |
| chr2.240000001.245000000 | 7.45E-03 | 1.99E-03 | 3.75 | 1.77E-04 | *** |
| chr20.1.5000000 | 5.27E-03 | 1.77E-03 | 2.98 | 2.91E-03 | ** |

| | | | | | |
|---|---|---|---|---|---|
| chr20.5000001.10000000 | 3.56E-03 | 2.00E-03 | 1.78 | 7.50E-02 | |
| chr20.10000001.15000000 | 2.56E-03 | 2.04E-03 | 1.26 | 2.09E-01 | |
| chr20.15000001.20000000 | 1.25E-02 | 2.22E-03 | 5.64 | 1.75E-08 | *** |
| chr20.20000001.25000000 | 4.93E-03 | 1.90E-03 | 2.60 | 9.43E-03 | ** |
| chr20.25000001.30000000 | 1.86E-03 | 7.23E-04 | 2.57 | 1.02E-02 | * |
| chr20.30000001.35000000 | 4.36E-04 | 1.02E-03 | 0.43 | 6.68E-01 | |
| chr20.35000001.40000000 | 5.15E-03 | 1.73E-03 | 2.98 | 2.92E-03 | ** |
| chr20.40000001.45000000 | 2.46E-03 | 1.64E-03 | 1.51 | 1.32E-01 | |
| chr20.45000001.50000000 | 7.97E-04 | 1.72E-03 | 0.46 | 6.43E-01 | |
| chr20.50000001.55000000 | 6.20E-03 | 1.91E-03 | 3.24 | 1.21E-03 | ** |
| chr20.55000001.60000000 | 4.40E-03 | 1.87E-03 | 2.35 | 1.87E-02 | * |
| chr20.60000001.65000000 | 4.65E-03 | 1.49E-03 | 3.13 | 1.78E-03 | ** |
| chr21.5000001.10000000 | 3.39E-04 | 1.93E-03 | 0.18 | 8.60E-01 | |
| chr21.10000001.15000000 | -9.92E-05 | 7.57E-04 | -0.13 | 8.96E-01 | |
| chr21.15000001.20000000 | 3.38E-03 | 1.64E-03 | 2.06 | 3.95E-02 | * |
| chr21.20000001.25000000 | 6.07E-03 | 2.25E-03 | 2.70 | 6.95E-03 | ** |
| chr21.25000001.30000000 | 9.42E-03 | 2.25E-03 | 4.18 | 2.93E-05 | *** |
| chr21.30000001.35000000 | 4.68E-03 | 1.90E-03 | 2.46 | 1.40E-02 | * |
| chr21.35000001.40000000 | 6.71E-03 | 1.91E-03 | 3.53 | 4.26E-04 | *** |
| chr21.40000001.45000000 | 6.23E-03 | 2.16E-03 | 2.89 | 3.89E-03 | ** |
| chr21.45000001.50000000 | 1.62E-03 | 1.46E-03 | 1.11 | 2.67E-01 | |
| chr22.15000001.20000000 | 3.69E-03 | 1.22E-03 | 3.03 | 2.45E-03 | ** |
| chr22.20000001.25000000 | 3.71E-03 | 1.75E-03 | 2.13 | 3.33E-02 | * |
| chr22.25000001.30000000 | 7.19E-03 | 1.74E-03 | 4.13 | 3.74E-05 | *** |
| chr22.30000001.35000000 | 2.43E-03 | 1.88E-03 | 1.29 | 1.97E-01 | |
| chr22.35000001.40000000 | 4.07E-03 | 1.57E-03 | 2.60 | 9.39E-03 | ** |
| chr22.40000001.45000000 | 3.78E-04 | 1.64E-03 | 0.23 | 8.18E-01 | |
| chr22.45000001.50000000 | 5.13E-03 | 2.22E-03 | 2.31 | 2.09E-02 | * |
| chr22.50000001.55000000 | 5.78E-04 | 9.00E-04 | 0.64 | 5.21E-01 | |
| chr3.1.5000000 | 6.72E-03 | 1.94E-03 | 3.46 | 5.41E-04 | *** |
| chr3.5000001.10000000 | 6.18E-03 | 2.07E-03 | 2.98 | 2.86E-03 | ** |
| chr3.10000001.15000000 | 2.22E-03 | 2.03E-03 | 1.10 | 2.73E-01 | |
| chr3.15000001.20000000 | 3.82E-03 | 1.57E-03 | 2.43 | 1.51E-02 | * |
| chr3.20000001.25000000 | 8.03E-03 | 2.18E-03 | 3.69 | 2.28E-04 | *** |
| chr3.25000001.30000000 | 4.62E-03 | 1.67E-03 | 2.77 | 5.65E-03 | ** |

| | | | | | |
|---|---|---|---|---|---|
| chr3.30000001.35000000 | 4.70E-03 | 2.06E-03 | 2.28 | 2.29E-02 | * |
| chr3.35000001.40000000 | 3.65E-03 | 1.63E-03 | 2.24 | 2.50E-02 | * |
| chr3.40000001.45000000 | 5.35E-03 | 1.51E-03 | 3.56 | 3.79E-04 | *** |
| chr3.45000001.50000000 | -9.46E-04 | 1.49E-03 | -0.63 | 5.26E-01 | |
| chr3.50000001.55000000 | 1.85E-03 | 1.25E-03 | 1.48 | 1.40E-01 | |
| chr3.55000001.60000000 | 4.99E-03 | 1.70E-03 | 2.93 | 3.39E-03 | ** |
| chr3.60000001.65000000 | 3.05E-03 | 1.87E-03 | 1.63 | 1.03E-01 | |
| chr3.65000001.70000000 | -1.37E-04 | 2.10E-03 | -0.07 | 9.48E-01 | |
| chr3.70000001.75000000 | 7.90E-03 | 1.79E-03 | 4.41 | 1.04E-05 | *** |
| chr3.75000001.80000000 | 1.38E-03 | 1.76E-03 | 0.78 | 4.33E-01 | |
| chr3.80000001.85000000 | 2.84E-03 | 1.79E-03 | 1.58 | 1.14E-01 | |
| chr3.85000001.90000000 | 3.85E-03 | 1.99E-03 | 1.93 | 5.34E-02 | |
| chr3.90000001.95000000 | 2.53E-03 | 1.02E-03 | 2.48 | 1.30E-02 | * |
| chr3.95000001.100000000 | 2.48E-05 | 1.40E-03 | 0.02 | 9.86E-01 | |
| chr3.100000001.105000000 | 3.39E-03 | 1.67E-03 | 2.03 | 4.25E-02 | * |
| chr3.105000001.110000000 | 7.39E-04 | 1.45E-03 | 0.51 | 6.09E-01 | |
| chr3.110000001.115000000 | -1.28E-03 | 1.82E-03 | -0.71 | 4.80E-01 | |
| chr3.115000001.120000000 | 2.15E-03 | 1.79E-03 | 1.20 | 2.29E-01 | |
| chr3.120000001.125000000 | 3.06E-03 | 1.96E-03 | 1.56 | 1.19E-01 | |
| chr3.125000001.130000000 | 5.73E-03 | 1.89E-03 | 3.03 | 2.45E-03 | ** |
| chr3.130000001.135000000 | 8.23E-03 | 2.01E-03 | 4.09 | 4.44E-05 | *** |
| chr3.135000001.140000000 | 1.02E-03 | 1.51E-03 | 0.67 | 5.02E-01 | |
| chr3.140000001.145000000 | 1.98E-03 | 1.82E-03 | 1.09 | 2.78E-01 | |
| chr3.145000001.150000000 | 5.42E-03 | 1.97E-03 | 2.75 | 5.89E-03 | ** |
| chr3.150000001.155000000 | 7.07E-03 | 1.83E-03 | 3.86 | 1.15E-04 | *** |
| chr3.155000001.160000000 | -4.33E-04 | 1.70E-03 | -0.26 | 7.99E-01 | |
| chr3.160000001.165000000 | 2.18E-03 | 1.56E-03 | 1.39 | 1.64E-01 | |
| chr3.165000001.170000000 | 5.95E-03 | 1.56E-03 | 3.81 | 1.41E-04 | *** |
| chr3.170000001.175000000 | 4.57E-03 | 1.90E-03 | 2.40 | 1.64E-02 | * |
| chr3.175000001.180000000 | 2.88E-03 | 1.76E-03 | 1.64 | 1.01E-01 | |
| chr3.180000001.185000000 | 6.38E-03 | 1.67E-03 | 3.81 | 1.39E-04 | *** |
| chr3.185000001.190000000 | 2.38E-03 | 1.89E-03 | 1.26 | 2.08E-01 | |
| chr3.190000001.195000000 | 5.90E-03 | 2.44E-03 | 2.42 | 1.57E-02 | * |
| chr3.195000001.200000000 | 1.51E-03 | 8.66E-04 | 1.74 | 8.19E-02 | |
| chr4.1.5000000 | 2.35E-03 | 1.71E-03 | 1.38 | 1.68E-01 | |

| | | | | | |
|---|---|---|---|---|---|
| chr4.5000001.10000000 | 4.12E-03 | 1.95E-03 | 2.12 | 3.40E-02 | * |
| chr4.10000001.15000000 | 3.85E-03 | 1.71E-03 | 2.25 | 2.44E-02 | * |
| chr4.15000001.20000000 | 1.07E-02 | 2.01E-03 | 5.33 | 1.01E-07 | *** |
| chr4.20000001.25000000 | 5.72E-03 | 2.03E-03 | 2.82 | 4.88E-03 | ** |
| chr4.25000001.30000000 | 5.10E-03 | 1.64E-03 | 3.12 | 1.83E-03 | ** |
| chr4.30000001.35000000 | 4.79E-03 | 1.98E-03 | 2.42 | 1.54E-02 | * |
| chr4.35000001.40000000 | 7.16E-03 | 2.03E-03 | 3.52 | 4.29E-04 | *** |
| chr4.40000001.45000000 | -1.63E-04 | 1.69E-03 | -0.10 | 9.23E-01 | |
| chr4.45000001.50000000 | 6.46E-04 | 1.65E-03 | 0.39 | 6.96E-01 | |
| chr4.50000001.55000000 | 3.00E-03 | 1.28E-03 | 2.35 | 1.90E-02 | * |
| chr4.55000001.60000000 | 3.77E-03 | 1.93E-03 | 1.95 | 5.11E-02 | |
| chr4.60000001.65000000 | 4.85E-03 | 2.01E-03 | 2.41 | 1.60E-02 | * |
| chr4.65000001.70000000 | 6.08E-03 | 1.88E-03 | 3.24 | 1.22E-03 | ** |
| chr4.70000001.75000000 | 8.25E-03 | 2.04E-03 | 4.05 | 5.17E-05 | *** |
| chr4.75000001.80000000 | 4.18E-03 | 2.02E-03 | 2.07 | 3.86E-02 | * |
| chr4.80000001.85000000 | 1.10E-02 | 2.04E-03 | 5.39 | 7.09E-08 | *** |
| chr4.85000001.90000000 | 5.26E-03 | 1.88E-03 | 2.79 | 5.21E-03 | ** |
| chr4.90000001.95000000 | 4.97E-03 | 2.02E-03 | 2.46 | 1.38E-02 | * |
| chr4.95000001.100000000 | 2.33E-03 | 1.60E-03 | 1.46 | 1.46E-01 | |
| chr4.100000001.105000000 | 5.41E-03 | 1.81E-03 | 2.99 | 2.82E-03 | ** |
| chr4.105000001.110000000 | 2.92E-03 | 1.63E-03 | 1.79 | 7.40E-02 | |
| chr4.110000001.115000000 | 5.26E-03 | 2.11E-03 | 2.50 | 1.24E-02 | * |
| chr4.115000001.120000000 | 3.91E-03 | 1.79E-03 | 2.18 | 2.94E-02 | * |
| chr4.120000001.125000000 | 2.02E-03 | 1.68E-03 | 1.20 | 2.30E-01 | |
| chr4.125000001.130000000 | 2.99E-03 | 1.81E-03 | 1.65 | 9.87E-02 | |
| chr4.130000001.135000000 | 1.16E-02 | 2.22E-03 | 5.20 | 1.99E-07 | *** |
| chr4.135000001.140000000 | 7.41E-03 | 2.04E-03 | 3.64 | 2.70E-04 | *** |
| chr4.140000001.145000000 | 5.35E-04 | 1.56E-03 | 0.34 | 7.31E-01 | |
| chr4.145000001.150000000 | 4.57E-03 | 1.66E-03 | 2.75 | 6.01E-03 | ** |
| chr4.150000001.155000000 | 4.17E-03 | 1.70E-03 | 2.46 | 1.39E-02 | * |
| chr4.155000001.160000000 | 7.63E-03 | 1.87E-03 | 4.09 | 4.38E-05 | *** |
| chr4.160000001.165000000 | 5.88E-03 | 2.03E-03 | 2.90 | 3.79E-03 | ** |
| chr4.165000001.170000000 | 6.19E-03 | 1.78E-03 | 3.47 | 5.23E-04 | *** |
| chr4.170000001.175000000 | 3.39E-04 | 1.67E-03 | 0.20 | 8.39E-01 | |
| chr4.175000001.180000000 | 4.00E-03 | 2.15E-03 | 1.86 | 6.33E-02 | |

| | | | | | |
|---|---|---|---|---|---|
| chr4.180000001.185000000 | 4.39E-03 | 1.95E-03 | 2.25 | 2.42E-02 | * |
| chr4.185000001.190000000 | 6.26E-03 | 2.15E-03 | 2.91 | 3.61E-03 | ** |
| chr4.190000001.195000000 | -5.12E-04 | 9.36E-04 | -0.55 | 5.85E-01 | |
| chr5.1.5000000 | 1.14E-02 | 2.22E-03 | 5.15 | 2.73E-07 | *** |
| chr5.5000001.10000000 | 7.59E-03 | 2.43E-03 | 3.13 | 1.77E-03 | ** |
| chr5.10000001.15000000 | 2.02E-04 | 1.62E-03 | 0.12 | 9.01E-01 | |
| chr5.15000001.20000000 | 3.31E-03 | 1.79E-03 | 1.85 | 6.51E-02 | |
| chr5.20000001.25000000 | 6.67E-03 | 2.05E-03 | 3.25 | 1.17E-03 | ** |
| chr5.25000001.30000000 | 1.29E-03 | 1.96E-03 | 0.66 | 5.11E-01 | |
| chr5.30000001.35000000 | 6.66E-03 | 1.93E-03 | 3.45 | 5.69E-04 | *** |
| chr5.35000001.40000000 | 4.37E-03 | 1.75E-03 | 2.50 | 1.25E-02 | * |
| chr5.40000001.45000000 | 2.31E-03 | 1.60E-03 | 1.45 | 1.48E-01 | |
| chr5.45000001.50000000 | -9.63E-04 | 6.81E-04 | -1.42 | 1.57E-01 | |
| chr5.50000001.55000000 | 6.90E-04 | 1.85E-03 | 0.37 | 7.10E-01 | |
| chr5.55000001.60000000 | -7.50E-04 | 1.87E-03 | -0.40 | 6.88E-01 | |
| chr5.60000001.65000000 | 1.87E-03 | 1.65E-03 | 1.14 | 2.55E-01 | |
| chr5.65000001.70000000 | 7.03E-03 | 1.69E-03 | 4.16 | 3.21E-05 | *** |
| chr5.70000001.75000000 | 8.69E-03 | 1.92E-03 | 4.52 | 6.31E-06 | *** |
| chr5.75000001.80000000 | 6.42E-03 | 1.94E-03 | 3.30 | 9.60E-04 | *** |
| chr5.80000001.85000000 | 1.21E-03 | 1.49E-03 | 0.81 | 4.18E-01 | |
| chr5.85000001.90000000 | 4.21E-03 | 1.62E-03 | 2.59 | 9.53E-03 | ** |
| chr5.90000001.95000000 | 2.74E-03 | 1.49E-03 | 1.84 | 6.58E-02 | |
| chr5.95000001.100000000 | 4.27E-03 | 1.64E-03 | 2.60 | 9.41E-03 | ** |
| chr5.100000001.105000000 | 5.10E-03 | 1.62E-03 | 3.15 | 1.63E-03 | ** |
| chr5.105000001.110000000 | 5.65E-03 | 1.86E-03 | 3.04 | 2.38E-03 | ** |
| chr5.110000001.115000000 | 3.73E-03 | 1.78E-03 | 2.09 | 3.65E-02 | * |
| chr5.115000001.120000000 | 5.18E-03 | 1.69E-03 | 3.07 | 2.14E-03 | ** |
| chr5.120000001.125000000 | 2.06E-03 | 1.80E-03 | 1.15 | 2.52E-01 | |
| chr5.125000001.130000000 | 3.15E-03 | 1.83E-03 | 1.72 | 8.56E-02 | |
| chr5.130000001.135000000 | 4.19E-03 | 1.42E-03 | 2.95 | 3.20E-03 | ** |
| chr5.135000001.140000000 | 5.65E-03 | 1.59E-03 | 3.55 | 3.85E-04 | *** |
| chr5.140000001.145000000 | 2.54E-03 | 1.62E-03 | 1.57 | 1.17E-01 | |
| chr5.145000001.150000000 | 2.32E-03 | 1.87E-03 | 1.24 | 2.15E-01 | |
| chr5.150000001.155000000 | 3.39E-06 | 1.67E-03 | 0.00 | 9.98E-01 | |
| chr5.155000001.160000000 | 7.75E-04 | 1.83E-03 | 0.42 | 6.72E-01 | |

| | | | | | |
|---|---|---|---|---|---|
| chr5.160000001.165000000 | 1.69E-03 | 8.53E-04 | 1.98 | 4.79E-02 | * |
| chr5.165000001.170000000 | 1.17E-02 | 1.35E-03 | 8.67 | <2.2E-16 | *** |
| chr5.170000001.175000000 | -3.33E-04 | 1.73E-03 | -0.19 | 8.47E-01 | |
| chr5.175000001.180000000 | 6.34E-03 | 1.78E-03 | 3.56 | 3.68E-04 | *** |
| chr5.180000001.185000000 | -6.44E-04 | 6.80E-04 | -0.95 | 3.43E-01 | |
| chr6.1.5000000 | 1.02E-02 | 2.13E-03 | 4.81 | 1.56E-06 | *** |
| chr6.5000001.10000000 | 4.50E-03 | 1.81E-03 | 2.49 | 1.29E-02 | * |
| chr6.10000001.15000000 | 4.39E-03 | 2.12E-03 | 2.07 | 3.86E-02 | * |
| chr6.15000001.20000000 | 1.33E-03 | 1.83E-03 | 0.73 | 4.65E-01 | |
| chr6.20000001.25000000 | 8.67E-03 | 1.93E-03 | 4.48 | 7.48E-06 | *** |
| chr6.25000001.30000000 | 1.45E-03 | 1.27E-03 | 1.14 | 2.55E-01 | |
| chr6.30000001.35000000 | 3.52E-04 | 9.14E-04 | 0.39 | 7.00E-01 | |
| chr6.35000001.40000000 | 3.85E-03 | 1.71E-03 | 2.25 | 2.43E-02 | * |
| chr6.40000001.45000000 | 2.84E-03 | 1.52E-03 | 1.87 | 6.18E-02 | |
| chr6.45000001.50000000 | 4.83E-03 | 1.68E-03 | 2.88 | 3.98E-03 | ** |
| chr6.50000001.55000000 | 8.47E-03 | 1.84E-03 | 4.61 | 4.00E-06 | *** |
| chr6.55000001.60000000 | 1.79E-03 | 1.33E-03 | 1.34 | 1.79E-01 | |
| chr6.60000001.65000000 | 1.26E-03 | 1.31E-03 | 0.96 | 3.35E-01 | |
| chr6.65000001.70000000 | 5.08E-03 | 1.95E-03 | 2.61 | 9.16E-03 | ** |
| chr6.70000001.75000000 | 6.96E-03 | 1.92E-03 | 3.63 | 2.83E-04 | *** |
| chr6.75000001.80000000 | 2.51E-03 | 1.65E-03 | 1.52 | 1.29E-01 | |
| chr6.80000001.85000000 | 4.90E-03 | 1.84E-03 | 2.67 | 7.62E-03 | ** |
| chr6.85000001.90000000 | 1.40E-03 | 1.75E-03 | 0.80 | 4.23E-01 | |
| chr6.90000001.95000000 | 4.45E-03 | 1.92E-03 | 2.32 | 2.05E-02 | * |
| chr6.95000001.100000000 | 3.70E-03 | 1.88E-03 | 1.97 | 4.84E-02 | * |
| chr6.100000001.105000000 | 5.55E-03 | 2.25E-03 | 2.47 | 1.35E-02 | * |
| chr6.105000001.110000000 | 4.71E-03 | 1.62E-03 | 2.91 | 3.60E-03 | ** |
| chr6.110000001.115000000 | 6.08E-03 | 1.88E-03 | 3.24 | 1.21E-03 | ** |
| chr6.115000001.120000000 | 6.09E-03 | 1.75E-03 | 3.47 | 5.15E-04 | *** |
| chr6.120000001.125000000 | 5.83E-03 | 2.19E-03 | 2.67 | 7.71E-03 | ** |
| chr6.125000001.130000000 | -3.38E-04 | 1.44E-03 | -0.23 | 8.15E-01 | |
| chr6.130000001.135000000 | 3.42E-03 | 1.59E-03 | 2.15 | 3.15E-02 | * |
| chr6.135000001.140000000 | 4.77E-03 | 1.74E-03 | 2.75 | 6.05E-03 | ** |
| chr6.140000001.145000000 | 4.00E-03 | 1.71E-03 | 2.34 | 1.96E-02 | * |
| chr6.145000001.150000000 | 3.83E-03 | 1.72E-03 | 2.22 | 2.62E-02 | * |

| | | | | | |
|---|---|---|---|---|---|
| chr6.150000001.155000000 | 7.47E-03 | 2.12E-03 | 3.52 | 4.30E-04 | *** |
| chr6.155000001.160000000 | 3.49E-03 | 1.89E-03 | 1.85 | 6.43E-02 | |
| chr6.160000001.165000000 | 6.15E-03 | 2.04E-03 | 3.01 | 2.60E-03 | ** |
| chr6.165000001.170000000 | 9.41E-03 | 2.53E-03 | 3.71 | 2.05E-04 | *** |
| chr6.170000001.175000000 | 8.84E-04 | 1.01E-03 | 0.88 | 3.79E-01 | |
| chr7.1.5000000 | 4.05E-03 | 1.70E-03 | 2.38 | 1.74E-02 | * |
| chr7.5000001.10000000 | 6.39E-03 | 1.88E-03 | 3.40 | 6.71E-04 | *** |
| chr7.10000001.15000000 | 6.23E-03 | 2.30E-03 | 2.70 | 6.91E-03 | ** |
| chr7.15000001.20000000 | 6.13E-03 | 2.18E-03 | 2.81 | 5.00E-03 | ** |
| chr7.20000001.25000000 | 6.75E-03 | 2.1 IE-03 | 3.20 | 1.38E-03 | ** |
| chr7.25000001.30000000 | 3.42E-03 | 2.00E-03 | 1.71 | 8.71E-02 | |
| chr7.30000001.35000000 | 6.75E-03 | 2.03E-03 | 3.33 | 8.63E-04 | *** |
| chr7.35000001.40000000 | 5.02E-03 | 2.19E-03 | 2.29 | 2.19E-02 | * |
| chr7.40000001.45000000 | -4.19E-05 | 1.72E-03 | -0.02 | 9.81E-01 | |
| chr7.45000001.50000000 | 4.50E-03 | 2.25E-03 | 2.00 | 4.54E-02 | * |
| chr7.50000001.55000000 | 4.09E-03 | 2.22E-03 | 1.84 | 6.53E-02 | |
| chr7.55000001.60000000 | 8.45E-04 | 1.52E-03 | 0.56 | 5.78E-01 | |
| chr7.60000001.65000000 | 1.51E-03 | 1.45E-03 | 1.04 | 2.98E-01 | |
| chr7.65000001.70000000 | 4.48E-03 | 1.73E-03 | 2.59 | 9.56E-03 | ** |
| chr7.70000001.75000000 | 2.72E-03 | 1.53E-03 | 1.77 | 7.68E-02 | |
| chr7.75000001.80000000 | 4.30E-03 | 1.83E-03 | 2.35 | 1.88E-02 | * |
| chr7.80000001.85000000 | 1.01E-02 | 2.15E-03 | 4.71 | 2.52E-06 | *** |
| chr7.85000001.90000000 | 5.78E-03 | 1.88E-03 | 3.08 | 2.06E-03 | ** |
| chr7.90000001.95000000 | 3.28E-03 | 1.79E-03 | 1.83 | 6.71E-02 | |
| chr7.95000001.100000000 | 1.39E-03 | 1.81E-03 | 0.77 | 4.42E-01 | |
| chr7.100000001.105000000 | 4.74E-03 | 1.72E-03 | 2.75 | 5.95E-03 | ** |
| chr7.105000001.110000000 | 2.37E-03 | 1.59E-03 | 1.48 | 1.38E-01 | |
| chr7.110000001.115000000 | 3.46E-03 | 1.66E-03 | 2.08 | 3.76E-02 | * |
| chr7.115000001.120000000 | 7.42E-04 | 1.69E-03 | 0.44 | 6.60E-01 | |
| chr7.120000001.125000000 | 5.24E-03 | 1.84E-03 | 2.85 | 4.40E-03 | ** |
| chr7.125000001.130000000 | 5.73E-03 | 1.61E-03 | 3.57 | 3.60E-04 | *** |
| chr7.130000001.135000000 | 4.10E-03 | 1.60E-03 | 2.57 | 1.02E-02 | * |
| chr7.135000001.140000000 | 4.81E-03 | 1.58E-03 | 3.04 | 2.36E-03 | ** |
| chr7.140000001.145000000 | 2.78E-03 | 1.54E-03 | 1.81 | 7.11E-02 | |
| chr7.145000001.150000000 | 2.43E-03 | 1.66E-03 | 1.46 | 1.43E-01 | |

| | | | | | |
|---|---|---|---|---|---|
| chr7.150000001.155000000 | 2.70E-03 | 2.02E-03 | 1.33 | 1.82E-01 | |
| chr7.155000001.160000000 | 4.52E-03 | 1.86E-03 | 2.43 | 1.50E-02 | * |
| chr8.1.5000000 | 9.32E-03 | 3.03E-03 | 3.08 | 2.08E-03 | ** |
| chr8.5000001.10000000 | 6.91E-03 | 2.36E-03 | 2.93 | 3.42E-03 | ** |
| chr8.10000001.15000000 | 8.08E-03 | 2.31E-03 | 3.50 | 4.63E-04 | *** |
| chr8.15000001.20000000 | 5.92E-03 | 2.42E-03 | 2.45 | 1.42E-02 | * |
| chr8.20000001.25000000 | 1.69E-03 | 2.06E-03 | 0.82 | 4.13E-01 | |
| chr8.25000001.30000000 | 1.03E-02 | 2.03E-03 | 5.09 | 3.70E-07 | *** |
| chr8.30000001.35000000 | 2.37E-03 | 1.76E-03 | 1.35 | 1.76E-01 | |
| chr8.35000001.40000000 | 3.67E-03 | 1.65E-03 | 2.23 | 2.56E-02 | * |
| chr8.40000001.45000000 | 3.61E-03 | 1.43E-03 | 2.53 | 1.14E-02 | * |
| chr8.45000001.50000000 | 4.98E-04 | 7.88E-04 | 0.63 | 5.28E-01 | |
| chr8.50000001.55000000 | 2.51E-03 | 1.47E-03 | 1.71 | 8.83E-02 | |
| chr8.55000001.60000000 | 1.02E-02 | 2.33E-03 | 4.37 | 1.25E-05 | *** |
| chr8.60000001.65000000 | 5.91E-03 | 2.16E-03 | 2.73 | 6.38E-03 | ** |
| chr8.65000001.70000000 | -7.25E-04 | 1.82E-03 | -0.40 | 6.91E-01 | |
| chr8.70000001.75000000 | 4.81E-03 | 1.99E-03 | 2.42 | 1.55E-02 | * |
| chr8.75000001.80000000 | 3.82E-03 | 1.80E-03 | 2.13 | 3.35E-02 | * |
| chr8.80000001.85000000 | 5.00E-03 | 2.07E-03 | 2.41 | 1.59E-02 | * |
| chr8.85000001.90000000 | 4.48E-03 | 1.53E-03 | 2.93 | 3.35E-03 | ** |
| chr8.90000001.95000000 | 6.27E-03 | 1.72E-03 | 3.64 | 2.75E-04 | *** |
| chr8.95000001.100000000 | 3.49E-03 | 1.83E-03 | 1.91 | 5.67E-02 | |
| chr8.100000001.105000000 | 3.84E-05 | 1.36E-03 | 0.03 | 9.78E-01 | |
| chr8.105000001.110000000 | 6.32E-03 | 1.69E-03 | 3.74 | 1.86E-04 | *** |
| chr8.110000001.115000000 | 4.73E-03 | 1.50E-03 | 3.16 | 1.57E-03 | ** |
| chr8.115000001.120000000 | 4.92E-03 | 1.92E-03 | 2.57 | 1.03E-02 | * |
| chr8.120000001.125000000 | 9.94E-03 | 1.97E-03 | 5.04 | 4.77E-07 | *** |
| chr8.125000001.130000000 | 6.63E-03 | 1.92E-03 | 3.46 | 5.36E-04 | *** |
| chr8.130000001.135000000 | 7.54E-03 | 2.10E-03 | 3.60 | 3.25E-04 | *** |
| chr8.135000001.140000000 | 1.10E-02 | 2.22E-03 | 4.95 | 7.44E-07 | *** |
| chr8.140000001.145000000 | 8.17E-03 | 2.09E-03 | 3.91 | 9.33E-05 | *** |
| chr8.145000001.150000000 | 1.42E-04 | 8.48E-04 | 0.17 | 8.67E-01 | |
| chr9.1.5000000 | 3.61E-03 | 2.01E-03 | 1.80 | 7.26E-02 | |
| chr9.5000001.10000000 | 1.14E-02 | 2.11E-03 | 5.37 | 8.09E-08 | *** |
| chr9.10000001.15000000 | 6.20E-03 | 1.92E-03 | 3.22 | 1.27E-03 | ** |

| | | | | | |
|---|---|---|---|---|---|
| chr9.15000001.20000000 | 3.60E-03 | 1.67E-03 | 2.16 | 3.12E-02 | * |
| chr9.20000001.25000000 | 4.85E-03 | 1.67E-03 | 2.90 | 3.78E-03 | ** |
| chr9.25000001.30000000 | 3.68E-03 | 1.76E-03 | 2.09 | 3.68E-02 | * |
| chr9.30000001.35000000 | 2.63E-03 | 1.91E-03 | 1.38 | 1.69E-01 | |
| chr9.35000001.40000000 | 1.93E-05 | 1.47E-03 | 0.01 | 9.90E-01 | |
| chr9.40000001.45000000 | -6.96E-04 | 7.81E-04 | -0.89 | 3.72E-01 | |
| chr9.45000001.50000000 | 9.09E-04 | 9.56E-03 | 0.10 | 9.24E-01 | |
| chr9.65000001.70000000 | 1.99E-03 | 9.65E-04 | 2.06 | 3.93E-02 | * |
| chr9.70000001.75000000 | 3.21E-03 | 1.76E-03 | 1.83 | 6.80E-02 | |
| chr9.75000001.80000000 | 2.49E-03 | 1.51E-03 | 1.65 | 9.91E-02 | |
| chr9.80000001.85000000 | 3.59E-03 | 1.76E-03 | 2.04 | 4.09E-02 | * |
| chr9.85000001.90000000 | 9.40E-03 | 1.89E-03 | 4.97 | 6.95E-07 | *** |
| chr9.90000001.95000000 | 6.17E-03 | 2.14E-03 | 2.89 | 3.84E-03 | ** |
| chr9.95000001.100000000 | 6.88E-03 | 1.90E-03 | 3.63 | 2.90E-04 | *** |
| chr9.100000001.105000000 | 2.92E-03 | 2.07E-03 | 1.41 | 1.59E-01 | |
| chr9.105000001.110000000 | 6.41E-03 | 1.93E-03 | 3.33 | 8.70E-04 | *** |
| chr9.110000001.115000000 | 3.41E-03 | 2.13E-03 | 1.60 | 1.10E-01 | |
| chr9.115000001.120000000 | 4.65E-03 | 2.09E-03 | 2.23 | 2.60E-02 | * |
| chr9.120000001.125000000 | 4.33E-03 | 1.65E-03 | 2.63 | 8.62E-03 | ** |
| chr9.125000001.130000000 | 5.95E-03 | 1.84E-03 | 3.23 | 1.25E-03 | ** |
| chr9.130000001.135000000 | 4.60E-03 | 1.52E-03 | 3.03 | 2.48E-03 | ** |
| chr9.135000001.140000000 | 2.24E-03 | 1.73E-03 | 1.29 | 1.97E-01 | |
| chr9.140000001.145000000 | 9.77E-05 | 6.93E-04 | 0.14 | 8.88E-01 | |

**Claims**

1. A method for determining a fetal nucleic acid concentration in maternal blood, comprising:

   step 1 of determining, by a processor, first genotype information based on alignment of sequencing data with at least a part of a reference genome, wherein the sequencing data are derived from a nucleic acid sample of the maternal blood;
   step 2 of correcting, by the processor, the first genotype information based on reference data by using a linkage disequilibrium relationship, to obtain second genotype information; and
   step 3 of determining, by the processor, the fetal nucleic acid concentration based on a difference between the first genotype information and the second genotype information.

2. The method according to claim 1, wherein the sequencing data are obtained by sequencing the nucleic acid sample of the maternal blood.

3. The method according to claim 1, wherein the reference genome comprises at least one strong linkage region in a human genome.

4. The method according to claim 3, wherein the strong linkage region has a length ranging from 5mb to 10 mb.

5. The method according to claim 1, wherein the first genotype information is determined based on a support number of sequencing reads.

6. The method according to claim 1, wherein the first genotype information comprises at least one of SNP or Indel.

7. The method according to claim 1, wherein the reference data comprise a plurality of pieces of variation site information and a plurality of pieces of variation frequency information.

8. The method according to claim 1, wherein the correcting is performed through IMPUTE2.

9. The method according to claim 1, wherein the step 3 further comprises:

   step 3-1 of determining a difference ratio between the first genotype information and the second genotype information; and
   step 3-2 of determining the fetal nucleic acid concentration in accordance with the difference ratio obtained in the step 3-1 and a pre-trained formula, wherein the pre-trained formula is determined based on a plurality of training samples with known fetal nucleic acid concentrations.

10. A device for determining a fetal nucleic acid concentration in maternal blood, comprising:

   an alignment unit configured to determine a first genotype information based on alignment between sequencing data and at least a part of a reference genome, wherein the sequencing data are derived from a nucleic acid sample of the maternal blood;
   a correction unit connected to the alignment unit and configured to correct the first genotype information based on reference data by using a linkage disequilibrium relationship to obtain second genotype information; and
   a calculation unit connected to the alignment unit and the correction unit, the calculation unit being configured to determine the fetal nucleic acid concentration based on a difference between the first genotype information and the second genotype information.

11. The device according to claim 10, wherein the sequencing data are obtained by sequencing the nucleic acid sample of the maternal blood, and/or

   the first genotype information is determined based on a support number of sequencing reads, and/or
   the first genotype information comprises at least one of SNV or Indel, and/or
   the reference data comprise a plurality of pieces of variation site information and a plurality of pieces of variation frequency information, and/or
   the correction is performed through IMPUTE2.

12. The device according to claim 10, wherein the reference genome comprises at least one strong linkage region in a human genome, preferably, the strong linkage region has a length ranging from 5mb to 10 mb.

13. The device according to claim 10, wherein the calculation unit further comprises:

   a difference ratio calculation unit configured to determine a difference ratio between the first genotype information and the second genotype information; and
   a fetal nucleic acid concentration calculation unit connected to the difference ratio calculation unit and configured to determine the fetal nucleic acid concentration in accordance with the difference ratio obtained in the difference ratio calculation unit and a pre-trained formula, wherein the pre-trained formula is determined based on a plurality of training samples with known fetal nucleic acid concentrations.

14. A computer device, comprising a memory, a processor, and a computer program stored on the memory and executable on the processor,
   wherein the program, when executed by the processor, implements the method according to any one of claims 1 to 9.

15. A computer readable storage medium having a computer program stored thereon, wherein the program, when executed by a processor, implements the method according to any one of claims 1 to 9.

**Patentansprüche**

1. Verfahren zum Bestimmen einer fetalen Nukleinsäurekonzentration in mütterlichem Blut, umfassend:

   Schritt 1 zum Bestimmen, durch einen Prozessor, von ersten Genotypinformationen basierend auf einer Ausrichtung von Sequenzierungsdaten mit mindestens einem Teil eines Referenzgenoms, wobei die Sequenzierungsdaten von einer Nukleinsäureprobe des mütterlichen Bluts abstammen;
   Schritt 2 zum Korrigieren, durch den Prozessor, der ersten Genotypinformationen basierend auf Referenzdaten durch Verwenden einer Verknüpfungsungleichgewichtsbeziehung, um zweite Genotypinformationen zu erhalten; und
   Schritt 3 zum Bestimmen, durch den Prozessor, der fetalen Nukleinsäurekonzentration basierend auf einer Differenz zwischen den ersten Genotypinformationen und den zweiten Genotypinformationen.

2. Verfahren nach Anspruch 1, wobei die Sequenzierungsdaten durch Sequenzieren der Nukleinsäureprobe des mütterlichen Bluts erhalten werden.

3. Verfahren nach Anspruch 1, wobei das Referenzgenom mindestens einen starken Verknüpfungsbereich in einem menschlichen Genom umfasst.

4. Verfahren nach Anspruch 3, wobei der starke Verknüpfungsbereich eine Länge aufweist, die von 5 mb bis 10 mb reicht.

5. Verfahren nach Anspruch 1, wobei die ersten Genotypinformationen basierend auf einer Unterstützungsanzahl von Sequenzierungs-Reads bestimmt werden.

6. Verfahren nach Anspruch 1, wobei die ersten Genotypinformationen mindestens eines von SNP oder Indel umfassen.

7. Verfahren nach Anspruch 1, wobei die Referenzdaten eine Vielzahl von Teilen von Variationsstelleninformationen und eine Vielzahl von Teilen von Variationsfrequenzinformationen umfassen.

8. Verfahren nach Anspruch 1, wobei das Korrigieren mittels IMPUTE2 durchgeführt wird.

9. Verfahren nach Anspruch 1, wobei der Schritt 3 ferner umfasst:

   Schritt 3 bis 1 zum Bestimmen eines Differenzverhältnisses zwischen den ersten Genotypinformationen und den zweiten Genotypinformationen; und
   Schritt 3 bis 2 zum Bestimmen der fetalen Nukleinsäurekonzentration gemäß dem in Schritt 3 bis 1 erhaltenen Differenzverhältnis und einer vortrainierten Formel, wobei die vortrainierte Formel basierend auf einer Vielzahl von Trainingsproben mit bekannten fetalen Nukleinsäurekonzentrationen bestimmt wird.

10. Vorrichtung zum Bestimmen einer fetalen Nukleinsäurekonzentration in dem mütterlichen Blut, umfassend:

    eine Ausrichtungseinheit, die konfiguriert ist, um erste Genotypinformationen basierend auf der Ausrichtung zwischen Sequenzierungsdaten und mindestens einem Teil eines Referenzgenoms zu bestimmen, wobei die Sequenzierungsdaten von einer Nukleinsäureprobe des mütterlichen Bluts abstammen;
    eine Korrektureinheit, die mit der Ausrichtungseinheit verbunden und konfiguriert ist, um die ersten Genotypinformationen basierend auf Referenzdaten durch Verwenden einer Verknüpfungsungleichgewichtsbeziehung zu korrigieren, um zweite Genotypinformationen zu erhalten; und
    eine Berechnungseinheit, die mit der Ausrichtungseinheit und der Korrektureinheit verbunden ist, wobei die Berechnungseinheit konfiguriert ist, um die fetale Nukleinsäurekonzentration basierend auf einer Differenz zwischen den ersten Genotypinformationen und den zweiten Genotypinformationen zu bestimmen.

11. Vorrichtung nach Anspruch 10, wobei die Sequenzierungsdaten durch Sequenzieren der Nukleinsäureprobe des mütterlichen Bluts erhalten werden, und/oder

    die ersten Genotypinformationen basierend auf einer Unterstützungsanzahl von Sequenzierungs-Reads bestimmt werden, und/oder
    die ersten Genotypinformationen mindestens eines von SNV oder Indel umfassen, und/oder

**EP 3 916 105 B1**

die Referenzdaten eine Vielzahl von Teilen von Variationsstelleninformationen und eine Vielzahl von Teilen von Variationsfrequenzinformationen umfassen, und/oder
die Korrektur mittels IMPUTE2 durchgeführt wird.

**12.** Vorrichtung nach Anspruch 10, wobei das Referenzgenom mindestens einen starken Verknüpfungsbereich in einem menschlichen Genom umfasst, wobei vorzugsweise der starke Verknüpfungsbereich eine Länge aufweist, die von 5 mb bis 10 mb reicht.

**13.** Vorrichtung nach Anspruch 10, wobei die Berechnungseinheit ferner umfasst:

eine Differenzverhältnisberechnungseinheit, die konfiguriert ist, um ein Differenzverhältnis zwischen den ersten Genotypinformationen und den zweiten Genotypinformationen zu bestimmen; und
eine Berechnungseinheit für fetale Nukleinsäurekonzentration, die mit der Differenzverhältnisberechnungseinheit verbunden und konfiguriert ist, um die fetale Nukleinsäurekonzentration gemäß dem Differenzverhältnis, das in der Differenzverhältnisberechnungseinheit erhalten wird, und einer vortrainierten Formel zu bestimmen, wobei die vortrainierte Formel basierend auf einer Vielzahl von Trainingsproben mit bekannten fetalen Nukleinsäurekonzentrationen bestimmt wird.

**14.** Computervorrichtung, umfassend einen Speicher, einen Prozessor und ein Computerprogramm, das auf dem Speicher gespeichert und auf dem Prozessor ausführbar ist,
wobei das Programm, wenn es durch den Prozessor ausgeführt wird, das Verfahren nach einem der Ansprüche 1 bis 9 implementiert.

**15.** Computerlesbares Speichermedium, das ein darauf gespeichertes Computerprogramm aufweist, wobei das Programm, wenn es durch einen Prozessor ausgeführt wird, das Verfahren nach einem der Ansprüche 1 bis 9 implementiert.

**Revendications**

**1.** Procédé pour déterminer une concentration d'acide nucléique foetal dans le sang maternel, comprenant : l'étape 1 de détermination, par un processeur, de premières informations de génotype sur la base de l'alignement de données de séquençage avec au moins une partie d'un génome de référence, dans lequel les données de séquençage sont dérivées d'un échantillon d'acide nucléique du sang maternel ;

l'étape 2 de correction, par le processeur, des premières informations de génotype sur la base de données de référence en utilisant une relation de déséquilibre de liaison, pour obtenir des secondes informations de génotype ; et
l'étape 3 de détermination, par le processeur, de la concentration d'acide nucléique foetal sur la base d'une différence entre les premières informations de génotype et les secondes informations de génotype.

**2.** Procédé selon la revendication 1, dans lequel les données de séquençage sont obtenues en séquençant l'échantillon d'acide nucléique du sang maternel.

**3.** Procédé selon la revendication 1, dans lequel le génome de référence comprend au moins une région de liaison forte dans un génome humain.

**4.** Procédé selon la revendication 3, dans lequel la région de liaison forte a une longueur allant de 5 mb à 10 mb.

**5.** Procédé selon la revendication 1, dans lequel les premières informations de génotype sont déterminées sur la base d'un nombre de support de lectures de séquençage.

**6.** Procédé selon la revendication 1, dans lequel les premières informations de génotype comprennent au moins un de SNP ou d'Indel.

**7.** Procédé selon la revendication 1, dans lequel les données de référence comprennent une pluralité d'éléments d'information de sites de variation et une pluralité d'éléments d'information de fréquence de variation.

**8.** Procédé selon la revendication 1, dans lequel la correction est réalisée par IMPUTE2.

**9.** Procédé selon la revendication 1, dans lequel l'étape 3 comprend, en outre :

l'étape 3-1 de détermination d'un rapport de différence entre les premières informations de génotype et les secondes informations de génotype ; et

l'étape 3-2 de détermination de la concentration d'acide nucléique foetal en fonction du rapport de différence obtenu à l'étape 3-1 et d'une formule pré-entraînée, dans laquelle la formule pré-entraînée est déterminée sur la base d'une pluralité d'échantillons d'entraînement avec des concentrations d'acide nucléique foetal connues.

**10.** Dispositif pour déterminer une concentration d'acide nucléique foetal dans le sang maternel, comprenant :

une unité d'alignement configurée pour déterminer des premières informations de génotype sur la base de l'alignement entre les données de séquençage et au moins une partie d'un génome de référence, dans laquelle les données de séquençage sont dérivées d'un échantillon d'acide nucléique du sang maternel ;

une unité de correction connectée à l'unité d'alignement et configurée pour corriger les premières informations de génotype sur la base de données de référence en utilisant une relation de déséquilibre de liaison pour obtenir des secondes informations de génotype ; et

une unité de calcul connectée à l'unité d'alignement et à l'unité de correction, l'unité de calcul étant configurée pour déterminer la concentration d'acide nucléique foetal sur la base d'une différence entre les premières informations de génotype et les secondes informations de génotype.

**11.** Dispositif selon la revendication 10, dans lequel les données de séquençage sont obtenues en séquençant l'échantillon d'acide nucléique du sang maternel, et / ou

les premières informations de génotype sont déterminées sur la base d'un nombre de support de lectures de séquençage, et / ou

les premières informations de génotype comprennent au moins l'un de SNV ou d'Indel, et / ou

les données de référence comprennent une pluralité d'éléments d'information de sites de variation et une pluralité d'éléments d'information de fréquence de variation, et / ou la correction est effectuée par IMPUTE2.

**12.** Dispositif selon la revendication 10, dans lequel le génome de référence comprend au moins une région de liaison forte dans un génome humain, de préférence, la région de liaison forte a une longueur allant de 5 mb à 10 mb.

**13.** Dispositif selon la revendication 10, dans lequel l'unité de calcul comprend, en outre :

une unité de calcul de rapport de différence configurée pour déterminer un rapport de différence entre les premières informations de génotype et les secondes informations de génotype ; et

une unité de calcul de la concentration d'acide nucléique foetal connectée à l'unité de calcul du rapport de différence et configurée pour déterminer la concentration d'acide nucléique foetal selon le rapport de différence obtenu dans l'unité de calcul du rapport de différence et une formule pré-entraînée, dans laquelle la formule pré-entraînée est déterminée sur la base d'une pluralité d'échantillons d'entraînement avec des concentrations d'acide nucléique foetal connues.

**14.** Dispositif informatique, comprenant une mémoire, un processeur, et un programme informatique stocké sur la mémoire et exécutable sur le processeur,

dans lequel le programme, lorsqu'il est exécuté par le processeur, met en oeuvre le procédé selon l'une quelconque des revendications 1 à 9.

**15.** Support de stockage lisible par ordinateur sur lequel est stocké un programme informatique, dans lequel le programme, lorsqu'il est exécuté par un processeur, met en oeuvre le procédé selon l'une quelconque des revendications 1 à 9.

FIG. 1

FIG. 2

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• Genotype imputation in genome-wide association studies. **PORCU, E. ; SANNA, S. ; FUCHSBERGER, C. ; FRITSCHE, L.G.** Curr Protoc Hum Genet. 2013